(19) Europäisches Patentamt
European Patent Office
Office européen des brevets

(11) EP 2 042 513 A1

(12) **EUROPEAN PATENT APPLICATION**
published in accordance with Art. 153(4) EPC

(43) Date of publication:
**01.04.2009 Bulletin 2009/14**

(21) Application number: **07790899.4**

(22) Date of filing: **18.07.2007**

(51) Int Cl.:
***C07K 14/42*** (2006.01) ***A61K 38/00*** (2006.01)
***A61P 29/00*** (2006.01) ***A61P 35/00*** (2006.01)
***A61P 37/06*** (2006.01) ***A61P 37/08*** (2006.01)

(86) International application number:
**PCT/JP2007/064144**

(87) International publication number:
**WO 2008/013082 (31.01.2008 Gazette 2008/05)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **25.07.2006 JP 2006202305**

(71) Applicant: **Galpharma Co., Ltd.**
**Kagawa 761-0301 (JP)**

(72) Inventors:
- **NISHI, Nozomu,**
**GALPHARMA Co., Ltd.**
**Takamatsu-shi, Kagawa 7610301 (JP)**
- **HIRASHIMA, Mitsuomi**
**Takamatsu-shi, Kagawa 7618071 (JP)**
- **YAMAUCHI, Akira,**
**GALPHARMA Co., Ltd.**
**Takamatsu-shi, Kagawa 7610301 (JP)**

(74) Representative: **Helbing, Jörg**
**Patentanwälte**
**von Kreisler Selting Werner**
**Bahnhofsvorplatz 1**
**Deichmannhaus am Dom**
**50667 Köln (DE)**

(54) **GALECTIN 9-POLYMER CONJUGATE**

(57) Galectin 9, which is an immune function-controlling factor belonging to a new class that is different from known cytokines and is expected to be used as a therapeutic drug for various immune-related diseases such as an autoimmune disease, has disadvantages (such as high sensitivity to protease and poor solubility) to be solved in developing therapeutic drugs. Therefore, there is a need to overcome these disadvantages. Chemically modified galectin 9, prepared by conjugating a polymer (for example, poly(ethylene glycol: PEG) to galectin 9, in particular, stabilized galectin 9, is verified to exert higher solubility with improvements in pharmacodynamics and pharmacokinetics (for example, prolongation of action potential duration, such as prolongation of blood half-life and others) and in suppression of hemagglutination and thus has excellent characteristics as a drug.

【FIG. 7】

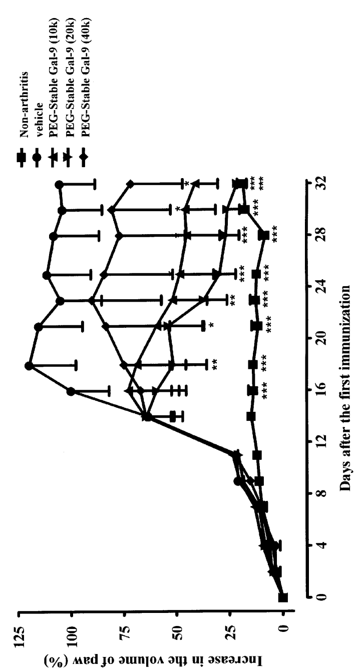


EP 2 042 513 A1

Printed by Jouve, 75001 PARIS (FR)

## Description

### FIELD OF THE INVENTION

The present invention relates to galectin 9-polymer conjugates. The present invention relates to chemically modified protein derivatives of galectin 9 and a modified galectin 9 protein (stabilized galectin 9).

### BACKGROUND OF THE INVENTION

Galectin has an affinity to β-galactoside and is a lectin family member containing a conserved domain in the primary sequence. At present, not less than 10 types of mammalian galectins have been found, and various bioactivities thereof, such as cell-cell adhesion or cell-extracellular matrix adhesion, cell activation, cell proliferation, and apoptosis, have been reported.

The group of the present inventors has succeeded in cloning of a human T cell-derived eosinophil chemoattractant and has found (Non-Patent Document 2) that the eosinophil chemoattractant is ecalectin, which is a variant of human galectin 9 reported by Tureci, et al (hGal9, Non-Patent Document 1). Furthermore, the group of the present inventors has demonstrated that ecalectin and Gal9 are identical substances and that there are three-types of human Gal9, i.e., short, medium, and long types depending on the length of the link peptide (Non-Patent Document 3). WO 04/064857 (Aug. 5, 2004) (Patent Document 1) discloses that a drug containing Gal9 can be expected as an anti-tumor agent (anticancer agent), an anti-allergy agent, an immunosuppressive agent, an anti-autoimmune disease agent, an anti-inflammatory agent, or an adrenocortical steroid hormone substitute. It has been also reported that Gal9 induces apoptosis in activated T cells. In addition, WO 05/093064 (Oct. 6, 2005) (Patent Document 2) discloses stabilized Gal9 (modified Gal9) and the use thereof.

Technology of conjugating biologically active protein with a polymer for suppressing the immune reaction, prolonging the circulation lifetime, or improving properties, such as water solubility and/or antigenicity, of the protein is applied to insulin, hemoglobin, interferon, interleukin-2, and the like, and the research and development of the technology are being continued at present using various types of protein. Typical examples thereof are conjugates prepared by coupling a peptide or a polypeptide to a poly(ethylene glycol) (PEG) or a watersoluble polymer similar thereto (Patent Document 3).

However, in many biologically active substances, the bioactivity is significantly reduced or lost by conjugation, and this problem is recognized to be still difficult to be solved. Also, this problem is caused when an active site that is necessary to obtain pharmaceutical activity or a site that participates in biological activity is blocked by a polymer. In general, a conjugation reaction of a polymer and protein is conducted in a solution system. Accordingly, under present circumstances, the aforementioned problem is very difficult to avoid.

[Patent Document 1] WO 04/064857 (Aug. 5, 2004)
[Patent Document 2] WO 05/093064 (Oct. 6, 2005)
[Patent Document 3] Japanese Patent Publication No. 56-23587
[Non-Patent Document 1] Tureci O., et al., J. Biol. Chem., 1997, 272(10): 6416-22.
[Non-Patent Document 2] Matsumoto R., et al., J. Biol. Chem., 1998, 273: 16976-84.
[Non-Patent Document 3] Matsushita N., et al., J. Biol. Chem., 2000, 275: 8355-60.

### SUMMARY OF THE INVENTION

Galectin 9 is an immune function-controlling factor belonging to a new class that is different from that of known cytokines and is expected to be used as a therapeutic drug for various immune-related diseases such as an autoimmune disease. For development into therapeutic drugs, however, galectin 9 has disadvantages, such as high protease susceptibility and poor solubility, to be solved. In order to overcome these disadvantages owing to the properties of native galectin 9 itself, a modified galectin 9 protein, i.e., stabilized galectin 9, is designed and produced by modifying its linker peptide. The stabilized galectin 9 has, in comparison with the native type, the following properties:

(1) increased resistance to protease,
(2) increased bioactivity, and
(3) increased solubility.

However, further improvements are required when it is intended to use galectin 9 as a therapeutic drug. For example, further improvements are required in solubility, in in vivo pharmacodynamics and pharmacokinetics (for example, prolongation of action potential duration, such as prolongation of blood half-life), and in suppression of hemagglutination activity and others.

The present inventors have conducted intensive studies for solving the aforementioned drawbacks and have studied on bioconjugation, i.e., binding of an artificially synthesized and purified polymer to galectin 9 or a modified galectin 9 protein, for imparting various functions (such as targeting and slow releasing functions) to the polymer and imparting the properties provided with the polymer to a drug. As a result, the present inventors have succeeded in obtaining improved galectin 9 properties by chemically modifying, in particular, by one bioconjugation, i.e., PEGylation, of galectin 9, especially stabilized galectin 9. Thus, the present invention has been achieved.
The present invention provides the following:

(1) A chemically modified protein derivative of galectin 9 or a modified galectin 9 protein, which is a galectin 9-polymer conjugate wherein which a polymer is bound, through a covalent bond, to a member selected from the group consisting of galectin 9 and modified galectin 9 proteins;
(2) The chemically modified protein derivative according to the aforementioned (1), wherein the polymer is a polyalkylene oxide or an alkoxy-terminated polyalkylene oxide;
(3) The chemically modified protein derivative according to the aforementioned (1) or (2), wherein the polymer is a poly(ethylene glycol) (PEG) or a lower alkoxy-terminated poly(ethylene glycol);
(4) The chemically modified protein derivative according to any of the aforementioned (1) to (3), wherein the polymer is a monomethoxy-poly(ethylene glycol) (mPEG);
(5) The chemically modified protein derivative according to any of the aforementioned (1) to (4), wherein the polymer has a molecular weight of approximately 200 to 100000;
(6) The chemically modified protein derivative according to any of the aforementioned (1) to (5), wherein the polymer has a molecular weight of approximately 5000 to 40000;
(7) The chemically modified protein derivative according to any of the aforementioned (1) to (6), which is prepared by reacting mPEG-SPA or mPEG2-NHS with a member selected from the group consisting of galectin 9 and modified galectin 9 proteins;
(8) The chemically modified protein derivative according to any of the aforementioned (1) to (7), wherein the member selected from the group consisting of galectin 9 and modified galectin 9 proteins is G9NC(null);
(9) A drug, which comprises a chemically modified protein derivative of galectin 9 or a modified galectin 9 protein that is a galectin 9-polymer conjugate wherein a polymer is bound, through a covalent bond, to a member selected from the group consisting of galectin 9 and modified galectin 9 proteins;
(10) The drug according to the aforementioned (9), which comprises a mixture of positional isomers of a polymer conjugate of a member selected from the group consisting of galectin 9 and modified galectin 9 proteins;
(11) The drug according to the aforementioned (9) or (10), which serves as an agent selected from the group consisting of anti-tumor agents (anticancer agents), anti-allergy agents, immunosuppressive agents, anti-autoimmune disease agents, anti-inflammatory agents, and adrenocortical steroid hormone substitutes;
(12) A process for producing a chemically modified protein derivative of galectin 9 or a modified galectin 9 protein, which comprises the step of:

reacting a member selected from the group consisting of galectin 9 and modified galectin 9 proteins with a PEGylating reagent under conditions whereby a covalent bond for PEGylating protein is formed to provide a PEGylated galectin 9 conjugate;

(13) The process according to the aforementioned (12), wherein the PEGylating reagent is carbonyloxy-N-dicarboximide; and
(14) The process according to the aforementioned (12) or (13), wherein the PEGylating reagent is mPEG-SPA or mPEG2-NHS.

ADVANTAGEOUS PROFILES OF THE INVENTION

The resultant chemically modified derivatives of galectin 9 or a modified galectin 9 protein, i.e., galectin 9-polymer conjugates, obtained according to the present invention, can exert usefully higher solubility and more prolonged blood elimination half-life and enhanced cytotoxic (cell-damaging) activity against tumor cells, in particular, malignant tumor cells. The galectin 9-polymer conjugates can achieve minimizing the risk of adverse side effects, i.e., removal of hemagglutination activity, and can be also expected to be excellent in in vivo drug distribution. Therefore, the galectin 9-polymer conjugates are useful and excellent as drugs and physiologically active substances.
The above objects and other objects, features, advantages, and aspects of the present invention are readily apparent to those skilled in the art from the following disclosures. It should be understood, however, that the disclosures in the

specification including the following best modes of carrying out the invention, examples, and others are illustrating preferred embodiments of the present invention and given for purposes of illustration only. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

BEST MODES OF CARRYING OUT THE INVENTION

In the present invention, the galectin 9 portion of the galectin 9-polymer conjugate is derived from "galectin 9" (Gal9). The galectin 9 may include, for example, L-type galectin 9 (Gal9L), M-type galectin 9 (Gal9M), S-type galectin 9 (Gal9S), or modified galectin 9 proteins (modified galectin 9 muteins or modified galectin 9 variants) having a modified galectin 9 amino acid sequence, such as stabilized galectin 9 (for example, G9NC(null)). Galectin 9 includes those prepared or isolated/purified from various types of sources. For example, said galectin 9 includes native galectin 9 (native Gal9 or naturally-occurring Gal9), produced in human leukocytes or cultured cell lines having the ability of producing Gal9, and recombinant galectin 9 (rGal9), obtained by gene recombinant techniques wherein a gene encoding Gal9, derived from the aforementioned leukocytes or specific cultured cell line, is incorporated into animal cells or microorganisms such as Escherichia coli. Both Gal9 and rGal9 can be advantageously used. In addition, Gal9 may also include those obtained from transgenic animals. Examples of the transgenic animal include mouse, rat, rabbit, bovine, hog, goat, and sheep. The Gal9 may be a mixture of two or more types of galectin 9. Human Gal9 (hGal9) can be advantageously used from the viewpoint of antigenicity.

As used herein, the term "galectin 9" (Gal9) is typically native Gal9. For native Gal9, at present, long-type (L-type) galectin 9 (galectin 9 long isoform or long type galectin 9: Gal9L), medium-type (M-type) galectin 9 (galectin 9 medium isoform or medium type galectin 9: Gal9M), and short-type (S-type) galectin 9 (galectin 9 short isoform or short type galectin 9: Gal9S) have been reported. Gal9L is considered to be a molecule wherein its N-terminal domain (N-terminal carbohydrate recognition domain: NCRD) is linked to its C-terminal domain (C-terminal carbohydrate recognition domain: CCRD) via a deduced link peptide region of SEQ ID NO: 4, disclosed in WO 02/37114 A1, Gal9M is considered to be a molecule wherein its NCRD is linked to its CCRD via a deduced link peptide region of SEQ ID NO: 5 in WO 02/37114 A1, and Gal9S is considered to be a molecule wherein its NCRD is linked to its CCRD via a deduced link peptide region of SEQ ID NO: 6 in WO 02/37114 A1. Gal9M differs from Gal9L in that amino acid residues corresponding to the sequence of SEQ ID NO: 7 in WO 02/37114 A1 are deleted from the link peptide region of Gal9L. Gal9S differs from Gal9M in that amino acid residues corresponding to the sequence of SEQ ID NO: 8 in WO 02/37114 A1 are deleted from the link peptide region of Gal9M. That is, Gal9S differs from L-type galectin 9 in that amino acid residues corresponding to SEQ ID NO: 9 in WO 02/37114 A1 are deleted from the deduced link peptide region of Gal9L. The sequence shown as SEQ ID NO: 1 in WO 02/37114 A1 is a typical amino acid sequence of Gal9L, the sequence shown as SEQ ID NO: 2 in WO 02/37114 A1 is a typical amino acid sequence of Gal9M, and the sequence shown as SEQ ID NO: 3 in WO 02/37114 A1 is a typical amino acid sequence of Gal9S.

Herein, galectin 9 may be understood so as to include the aforementioned Gal9L, Gal9M, Gal9S, other naturally-occurring variants of such galectin 9 family, their artificial variants with mutations (i.e., deletions, additions, modifications, and/or insertions of one or more amino acid residues such as one or several amino acid residues), and those containing a partial domain or partial peptide fragment thereof. All of those disclosed in WO 04/064857 (Aug. 5, 2004) and J. Biol. Chem., 2000, 275(12): 8355-8360 may be included herein. Such galectin 9 may include native galectin 9 variants and further all of the "modified galectin 9 proteins" (modified galectin 9 muteins or modified galectin 9 variants), "modified galectin 9 polypeptides" (modified galectin 9 mutein polypeptides), and "modified galectin 9 protein drugs" disclosed in PCT/JP2005/006580 (filing date: March 29, 2005). A particularly preferred example is G9NC(null) [polypeptide encoded by a nucleotide sequence of SEQ ID NO: 1 and having an amino acid sequence of SEQ ID NO: 2 in PCT/JP2005/006580 (filing date: March 29, 2005)] prepared in Example 1 of PCT/JP2005/006580 (filing date: March 29, 2005). It has been recognized that some "modified galectin 9 proteins" and "modified galectin 9 polypeptides" have more stabilized characteristics, compared to those of native galectin 9, and such modified galectin 9 proteins or polypeptides are also referred to as "stabilized galectin 9" herein. The stabilized galectin 9 is typically the aforementioned G9NC(null), but is not limited thereto. Those showing similar characteristics may be included therein.

The therapeutic and/or preventive agents and the therapeutic and/or preventive treatments according to the present invention can be conducted in accordance with the disclosure of WO 04/064857 (Aug. 5, 2004) or PCT/JP2005/006580 (filing date: March 29, 2005), and the description therein is hereby incorporated by reference into the present disclosure.

BRIEF DESCRIPTION OF THE DRAWINGS

FIG. 1 shows the protein elution pattern of ion-exchange chromatography using a RESOURCE S column (GE Healthcare Bioscience Corp.) and the SDS-polyacrylamide gel electrophoretic results of each fraction for stabilized galectin 9 PEGylated using mPEG2-NHS with a molecular weight of 20000 (mPEG2-NHS (20K) PEGylated G9NC (null)), wherein the solid line indicates absorbance at 280 nm and the dotted line indicates electric conductivity.

FIG. 2 shows the proliferation-inhibiting activity assay results of PEGylated modified galectin 9 proteins (PEGylated stabilized galectin 9) against human prostate cancer cell line PC-3, wherein G9Null refers to G9NC(null), G9Null-PEG-5K-SE2 refers to the SE2 fraction of stabilized galectin 9 (G9NC(null)) PEGylated with mPEG-SPA having a molecular weight of 5000, and G9Null-PEG-10K-SE2 refers to the SE2 fraction of stabilized galectin 9 (G9NC(null)) PEGylated with mPEG2-NHS having a molecular weight of 10000.

FIG. 3 shows the proliferation-inhibiting activity assay results of PEGylated modified galectin 9 proteins (PEGylated stabilized galectin 9) against human prostate cancer cell line PC-3, wherein G9Null refers to G9NC(null), G9Null-PEG-20K-SE2 refers to the SE2 fraction of stabilized galectin 9 (G9NC(null)) PEGylated with mPEG2-NHS having a molecular weight of 20000, and G9Null-PEG-40K-SE2 refers to the SE2 fraction of stabilized galectin 9 (G9NC (null)) PEGylated with mPEG2-NHS having a molecular weight of 40000.

FIG. 4 shows the proliferation-inhibiting activity assay results of PEGylated modified galectin 9 proteins (PEGylated stabilized galectin 9) against human prostate cancer cell line PC-3, wherein G9Null-PEG-5K-SE2 refers to the SE2 fraction of stabilized galectin 9 (G9NC(null)) PEGylated with mPEG-SPA having a molecular weight of 5000, G9Null-PEG-5K-SE1 refers to the SE1 fraction of stabilized galectin 9 (G9NC(null)) PEGylated with mPEG-SPA having a molecular weight of 5000, G9Null-PEG-10K-SE2 refers to the SE2 fraction of stabilized galectin 9 (G9NC(null)) PEGylated with mPEG2-NHS having a molecular weight of 10000, and G9Null-PEG-10K-SE1 refers to the SE1 fraction of stabilized galectin 9 (G9NC(null)) PEGylated with mPEG2-NHS having a molecular weight of 10000.

FIG. 5 shows the proliferation-inhibiting activity assay results of PEGylated modified galectin 9 proteins (PEGylated stabilized galectin 9) against human prostate cancer cell line PC-3, wherein G9Null-PEG-20K-SE2 refers to the SE2 fraction of stabilized galectin 9 (G9NC(null)) PEGylated with mPEG2-NHS having a molecular weight of 20000, G9Null-PEG-20K-SE1 refers to the SE1 fraction of stabilized galectin 9 (G9NC(null)) PEGylated with mPEG2-NHS having a molecular weight of 20000, G9Null-PEG-40K-SE2 refers to the SE2 fraction of stabilized galectin 9 (G9NC (null)) PEGylated with mPEG2-NHS having a molecular weight of 40000, and G9Null-PEG-40K-SE2 refers to the SE1 fraction of stabilized galectin 9 (G9NC(null)) PEGylated with mPEG2-NHS having a molecular weight of 40000.

FIG. 6 shows the hemagglutination assay results of PEGylated stabilized galectin 9 on rabbit erythrocytes and human erythrocytes.

FIG. 7 shows the therapeutic efficacy of PEGylated stabilized galectin 9 on a rat CIA model.

In the present invention, "gene recombination technology" or "gene recombination techniques" can be used for not only constructing, acquiring, isolating, or sequencing of targeted nucleic acid molecules (polynucleotides) and peptides (polypeptides), but also creating and producing recombinant constructs thereof. The gene recombination techniques (including recombinant DNA techniques) as can be used herein include those known in the art, for example, the methods described in J. Sambrook, et al., "Molecular Cloning: A Laboratory Manual (2nd edition (1989) & 3rd edition (2001))", Cold Spring Harbor Laboratory Press, Cold Spring Harbor, New York; D. M. Glover, et al. ed., "DNA Cloning", 2nd ed., Vols. 1 to 4, (The Practical Approach Series), IRL Press, Oxford University Press (1995); Nippon Seikagakukai (Biochemical Society of Japan) ed., "Zoku-Seikagaku Jikken Kouza 1, Idenshi Kenkyuho II (Lectures on Biochemical Experiments 1, Methods for Gene Study II)", Tokyo Kagaku Dojin, (1986); Nippon Seikagaku Kai (Biochemical Society of Japan) ed., "Shin-Seikagaku Jikken Kouza 2, Kakusan III (Kumikae DNA Gijutsu) (New Lectures on Biochemical Experiments 2, Nucleic Acids III (Recombinant DNA Technology))", Tokyo Kagaku Dojin, (1992); M. J. Gait (Ed), Oligonucleotide Synthesis, IRL Press (1984); B. D. Hames and S. J. Higgins (Ed), Nucleic Acid Hybridization, A Practical Approach, IRL Press Ltd., Oxford, UK (1985); B. D. Hames and S. J. Higgins (Ed), Transcription and Translation: A Practical Approach (Practical Approach Series), IRL Press Ltd., Oxford, UK (1984); B. Perbal, A Practical Guide to Molecular Cloning (2nd Edition), John Wiley & Sons, New York (1988); J. H. Miller and M. P. Calos (Ed), Gene Transfer Vectors for Mammalian Cells, Cold Spring Harbor Laboratory, Cold Spring Harbor, New York (1987); R. J. Mayer and J. H. Walker (Ed), Immunochemical Methods in Cell and Molecular Biology, Academic Press, (1987); R. K. Scopes, et al. (Ed), Protein Purification: Principles and Practice (2nd Edition, 1987 & 3rd Edition, 1993), Springer-Verlag, N.Y.; D. M. Weir and C. C. Blackwell (Ed), Handbook of Experimental Immunology, Vols. 1, 2, 3 and 4, Blackwell Scientific Publications, Oxford, (1986); L. A. Herzenberg, et al. (Ed), Weir's Handbook of Experimental Immunology, Vols. 1, 2, 3

and 4, Blackwell Science Ltd. (1997); R. W. Ellis (Ed), Vaccines new approaches to immunological problems, Butterworth-Heinemann, London (1992); R. Wu ed., "Methods in Enzymology", Vol. 68 (Recombinant DNA), Academic Press, New York (1980); R. Wu, et al. (Ed), "Methods in Enzymology", Vol. 100 (Recombinant DNA, Part B) & 101 (Recombinant DNA, Part C), Academic Press, New York (1983); R. Wu, et al. (Ed), "Methods in Enzymology", Vol. 153 (Recombinant DNA, Part D), 154 (Recombinant DNA, Part E) & 155 (Recombinant DNA, Part F), Academic Press, New York (1987); J. H. Miller (Ed), "Methods in Enzymology", Vol. 204, Academic Press, New York (1991); R. Wu, et al. (Ed), "Methods in Enzymology", Vol. 218, Academic Press, New York (1993); S. Weissman (Ed), "Methods in Enzymology", Vol. 303, Academic Press, New York (1999); J. C. Glorioso, et al. (Ed), "Methods in Enzymology", Vol. 306, Academic Press, New York (1999); Jeremy Thorner, et al. (Ed), "Methods in Enzymology", Vols. 326 to 328, Academic Press, New York (2000); and David R. Engelke, et al. (Ed), "Methods in Enzymology", Vol. 392, Academic Press, New York (2005), etc., or by methods described in the references quoted therein, or substantially equivalent methods thereto or modified methods thereof, the disclosures of which are incorporated herein by reference (hereinafter, all such techniques or methods shall be referred to as "gene recombination technology").

The galectin 9 polypeptides or the modified galectin 9 polypeptides of the present invention can be produced using various prokaryotic or eukaryotic expression systems (i.e., host cell/expression vector combinations). Potential cell types of the host cells include bacteria, yeasts, insect cells, mammal cells, and plant cells, but are not limited thereto. In a case using Escherichia coli cells, such host cells include for example cells derived from an E. coli K12 and B strains. Examples thereof as can be used herein are NM533, XL1-Blue, C600, DH1, DH5, DH11S, DH12S, DH5α, DH10B, HB101, MC1061, JM109, STBL2, BL21(DE3)pLysS derived from E. coli B834 and others, in combination with an appropriate expression vector selected. Examples of yeast may include baker's yeast and fission yeast, such as genus Saccharomyces, genus Schizosaccharomyces, and genus Pichia, more specifically, Saccharomyces cerevisiae, Schizosaccharomyces pombe, and Pichia pastoris, etc. They can be used with an appropriate expression vector selected. Representatives of the expression vector include pBR322, pUC18, pUC19, pUC118, pUC119, pSP64, pSP65, pTZ-18R/-18U, pTZ-19R/-19U, pGEM-3, pGEM-4, pGEM-3Z, pGEM-4Z, pGEM-5Zf(-), pGEMEX-1 (Promega), pBC KS (Stratagene), pBC SK (Stratagene), pBluescript™ SK (Stratagene), pBluescript™ II SK (Stratagene), pBluescript™ II KS (Stratagene), pBS (Stratagene), pAS, pKK223-3 (Amersham Pharmacia Biotech), pMC1403, pMC931, pKC30, pRSET-B (Invitrogen), pSE280 (Invitrogen), pBTrp2 (Boehringer Mannheim), pBTac1 (Boehringer Mannheim), pBTac2 (Roche), pGEX (Amersham Biosciences), pQE series (QIAGEN), pET Expression System (Novagen), YEP13 (ATCC37115), YEp24 (ATCC37051), YCp50 (ATCC37419), pHS19, and pHS15, and further include Invitrogen vectors for Mammalian Expression (for example, BsdCassette™ Vectors, Epitope-Tagged pcDNA™ Vectors, Eukaryotic TA Expression Kit, Flp-In™ Expression Vectors and Kits, Flp-In™ T-REx™ Expression Vectors, FreeStyle™ 293 Expression System, GeneSwitch™ System, pBC1 Milk Expression Vector Kit, pBudCE4.1, pcDNA™ Gateway™ Vectors, pcDNA™3.1 Directional TOPO™ Expression Kit, pcDNA™3.1-E Echo™ Expression Vector Kit, pcDNA™3.1/V5-His TOPO™ Expression Kit, pcDNA™4/HisMax and pcDNA™4/HisMax TOPO™ TA Expression Kit, pcDNA™4/TO-E Echo™ Vector Expression Kit, pcDNA™6 BioEase™ Gateway™ Biotinylation System, pcDNA/V5-GW/D-TOPO™ Vectors, pCEP4 and pREP4, pDisplay™ Vector, pEF6/V5-His TOPO™ TA Expression Kit, pFRT/lacZeo and pFRT/lacZeo2, pSecTag2 and pSecTag2/Hygro, pShooter™ Vectors, pVAX™200-DEST Vector System, pVAX1, pZeoSV2, T-REx™ Gateway™ Vectors, T-REx™ System, Tag-On-Demand™ Technology, Untagged pcDNA™ Vectors, Vivid Colors™ pcDNA™ 6.2 Fluorescent Protein Gateway™ Destination Vectors, an ZeoCassette™ Vectors), Clontech vectors (for example, Adenoviral Expression Vectors, Creator™ System Vectors, IRES Bicistronic Expression Vectors, Living Colors™ Fluorescent Protein Vectors, MATCHMARJER Vectors, Retroviral Expression Vectors, Signal Transduction Vectors, Tet Expression System Vectors, BacPak Baculovirus Expression Vectors, and HAT Protein Expression System Vectors), HaloTag™ pHT2 Vector, pACT Vectors, pBIND Vectors, pCAT™3 Vectors, pCI Vectors, phRG Vectors, phRL Vectors (Promega Corp.), and Novagen vectors for protein expression (for example, pTriEX™ Multisystem Expression Vectors, pBiEX™ Multisystem Expression Vectors, pTandem™-1 Vector, and pTK-neo DNA, and others), etc. Furthermore, other vectors that are known in the art or commercially available can be suitably selected and used.

Target products contained in cells (including culture cells), culture supernatants, or extracts can be purified by suitable combinations of widely known techniques for separation, isolation and purification. Such widely known techniques are , for example, salting-out such as ammonium sulfate precipitation; gel filtration on, for example, Sephadex™, etc.; ion exchange chromatography using carriers having, for example, a diethylaminoethyl or carboxymethyl group; hydrophobic chromatography using carriers having, for example, a hydrophobic group such as butyl, octyl, or phenyl, etc.; dye-gel (or chromophore-linked gel) chromatography; electrophoresis; dialysis; ultrafiltration; affinity chromatography; or high-performance liquid chromatography (HPLC); etc. Preferably, the target products can be isolated/purified by treatment with polyacrylamide gel electrophoresis (PAGE) or affinity chromatography in which ligands are immobilized. Said ligand may comprise antibodies including monoclonal antibodies or fragments thereof, capable of recognizing specific targets, lectins, saccharides, one member of a binding pair, and others. Examples of such techniques also include immunoaffinity chromatography, gelatin-agarose affinity chromatography, heparin-agarose chromatography, lactose-agarose chromatography, and other chromatography using a carrier such as ligand-immobilized agarose wherein

β-galactoside is immobilized as a ligand. In particular, the target products may be produced as fusion proteins or fusion polypeptides with gene recombination techniques, which can be simply purified by affinity chromatography utilizing a specifically binding ligand, such as an antibody to the fusion domain (tag), etc.

Galectin 9 and modified proteins thereof may be chemically synthesized. The techniques known in the peptide synthesis field, including, for example, chemical synthesis such as liquid phase synthesis and solid phase synthesis can be used for the synthesis of the proteins and peptide fragments thereof. In such techniques, using, for example, a resin for synthesis of proteins or peptides, appropriately protected amino acid is sequentially attached to the desired amino acid sequence on the resin according to various condensation methods as known in the art. Various activating reagents as known in the art are preferably used for the condensation reactions. For example, preferable activating reagents used herein include carbodiimides such as dicyclohexylcarbodiimide. A target product can be obtained by appropriately removing a protecting group when the product has the protecting group. Similarly, a nucleic acid encoding one member selected from the group consisting of galectin 9 and modified galectin 9 proteins may be chemically synthesized. For example, the nucleic acid can be chemically synthesized by a method such as a phosphotriester method, a phosphodiester method, a phosphite method, a phosphoamidite method, or a phosphonate method. It is generally known that the synthesis can be conveniently conducted on a modified solid-phase. For example, automated synthesizers can be used, and such synthesizers are commercially available. The sequence of a nucleic acid encoding one member selected from the group consisting of galectin 9 and modified galectin 9 proteins may comprise one ore more members selected from convenient or useful modifications, including, for example, codon modifications (for example, codon substitutions) suited for suitable expression in host cells selected; restriction sites; expression control sequences; regulatory sequences for facilitating the expression of a target gene; linkers, adapters, or the like, which are suitable for manipulating a target gene; sequences for controlling antibiotic resistance, controlling auxotrophy/metabolism, or useful for selection/detection, and others. The method of preparing galectin 9 for conjugation according to the present invention is not limited to those described herein.

In the present invention, the polymer portion of the galectin 9-polymer conjugate is derived from a "watersoluble polymer". The polymer furnishing the polymer portion include, for example, polyalkylene oxide, phospholipid polymers, polyvinylpyrrolidone, and polysaccharides such as dextran. Polymers that are watersoluble at room temperature are widely known, and those selected from such polymers can be used. Typical examples thereof include polyalkylene oxide homopolymers, polyoxyethylenated polyols, copolymers thereof, and block copolymers thereof. The polyalkylene oxide may include polyethylene glycol (PEG), polypropylene glycol, etc. Preferable PEG copolymers as can be utilized herein are non-branched molecules, for example, straight-chain type molecules, and also those non-substituted. It is, however, preferable to use those being substituted by an alkyl group at one end. The alkyl group present at the one end may be a $C_1$-$C_5$ alkyl group, preferably a $C_1$-$C_3$ alkyl group, and more preferably a methyl group.

The polyoxyethylenated polyol may include polyoxyethylenated glycerol, polyoxyethylenated sorbitol, and polyoxyethylenated glucose. The polyoxyethylenation (PEGylation) in polyoxyethylenated glycerol may include, for example, mono-PEGylation, di-PEGylation, and tri-PEGylation.

Preferred polymers include non-substituted PEG homopolymers, PEG homopolymers having a monomethyl substituent at one end (methoxypolyethylene glycol: mPEG), PEGylated glycerol, etc. The most preferred polymer is mPEG.

The polymer is not limited to those having specific molecular weights, as long as a desired target goal can be achieved. Typically, those polymers having a number average molecular weight ranging from about 200 to 100000 can be generally selected. The selected polymers can be those having a number average molecular weight ranging from about 500 to 90000, from about 1000 to 80000, from about 2000 to 70000, from about 3000 to 60000, from about 4000 to 50000, from about 4500 to 45000, or from about 5000 to 40000. Furthermore, in a case that the polymer is mPEG, its number average molecular weight is ranging, for example, from about 2500 to 7500, or from about 4000 to 6000. In another case, the selected species are those having a number average molecular weight ranging from about 8000 to 15000 or from about 9000 to 12000. Furthermore, in a case of another object, the selected species are those having a number average molecular weight ranging from about 15000 to 25000, or from about 18000 to 22000, and in another example, those having a number average molecular weight ranging from about 25000 to 50000, or from about 30000 to 45000.

Between the polymer portion and the galectin 9 protein portion, a direct linkage through a covalent bond may be positioned or a link is located through one member derived from coupling agents or linkers or a spacer (or spacer arm) selected from those forming suitable interrelationship between galectin 9 and the polymer. The spacer includes those linking covalently between the polymer portion and the galectin 9 portion. The binding between the polymer portion and the galectin 9 portion may include a bond selected from the group consisting of a carbon-carbon bond, a carbon-oxygen bond, a carbon-sulfur bond, a carbon-nitrogen bond, a sulfur-sulfur bond, and other bonds. Said binding can have one member selected from the group consisting of, for example, an ester bond (-CO-O-), a thioester bond, an amide bond (-NH-CO-), an ether bond, a thioether bond, a disulfide bond (-S-S-), an urethane bond (-O-CO-NH-), and others.

In order to form a conjugate by attaching a polymer, such as mPEG, to galectin 9, in general, one hydroxyl group of the polymer (for example, in mPEG or the like, one hydroxyl group at one terminal end) is exchanged with a reactive functional

group so as to enable conjugation to occur. In this exchange process is called, in many cases, activation. The product obtained by such activation is called an activated polymer or a functionalized polymer. In another process, one hydroxyl group of the polymer (for example, in mPEG or the like, one hydroxyl group at one terminal end) may be exchanged with an amino group or a sulfhydril group (HS-group), and then activation may be performed to give a corresponding activated polymer. In addition, activated PEG is called a PEGylating reagent or a PEGylation reagent (or a PEG reagent), in this field.

In order to form a bond to an activated polymer at the nitrogen atom site in the ε-amino group of a lysine residue or the N-terminal amino group present on protein, the group that reacts with the nitrogen atom of the protein, i.e., the activated group (functional group) of the polymer, is preferably a terminal carbonyloxy-N-dicarboximide group such as a succinimidyloxycarbonyl group, and others. The N-dicarboximide group may include an N-succinimide group, an N-phthalimide group, an N-glutarimide group, an N-tetrahydrophthalimide group, an N-5-norbornene-2,3-dicarboximide group, etc. The preferable group as can be used is an N-succinimide group. The terminal carbonyloxy-N-dicarboximide group can be formed by various known methods, for example, by a method of reacting a carbonyl halogenated compound or a carbonyl oxysulfonyl compound with N-hydroxydicarboximide such as N-hydroxysuccinimide. The N-hydroxydicarboximide includes N-hydroxysuccinimde, N-hydroxyphthalimide, N-hydroxyglutarimide, N-hydroxytetra-hydrophthalimide, N-hydroxy-5-norbornene-2,3-dicarboximide, etc.

The polymer may be treated with a coupling agent so as to form an activated carboxylic acid residue, as being present on, for example, carbonyl halogenated compounds or carbonyl oxysulfonyl compounds, between (a) the activated group (functional group; for example, terminal carbonyloxy-N-dicarboximide) of the polymer side that reacts with the aforementioned protein nitrogen atom site and (b) its polymer backbone or basic skeleton (for example, in a case where the polymer is PEG or mPEG, the repeating structural unit of $(-CH_2-CH_2-O-)$ or the $(-CH_2-CH_2-NH-)$ unit at one terminal end adjacent to the repeating structural unit of $(-CH_2-CH_2-O-)$). The polymer that reacts with aforementioned protein nitrogen atom site may be modified so as to facilitate the treatment with the coupling agent advantageously and/or form the appropriate interrelationship between the galectin 9 part and the polymer part. The polymer may be thereby provided with a spacer. The bridge that is present between the polymer backbone and galectin 9 can be called a spacer or a spacer-arm. The spacer may be a saturated or unsaturated hydrocarbon group that may include a nitrogen atom, an oxygen atom, a sulfur atom, or the like; may be either a straight-chain or branched-chain; and may provide a divalent binding group, a trivalent binding group, a trivalent binding group, or other multivalent groups. The spacer may be selected from those known to a person skilled in the art pertaining to the protein modification art and modified derivatives thereof. A suitable spacer can be appropriately selected. Typical spacers include those linked with a methylene chain, those derived from an amino acid such as lysine, those derived from an oligopeptide such as dipeptide, those derived from difunctional carboxylic anhydride such as succinic anhydride and glutaric anhydride, those derived from hydroxycarboxylic acid such as hydroxybutyric acid, and the like. Examples of the spacer are $-O-CH_2CH_2-CO-$, $-O-CH_2-CH(CH_3)-CO-$, $(-NH-CO-O-CH_2)_2CH-$, $-O-CH_2-CO-$, $-O-CO-NH-CH_2-CH_2-CO-$, $-O-CO-NH-CH_2-CH(CH_3)-CO-$, $-O-CO-NH-CH_2-CH_2-CH_2-CH_2-CH(CO-)-CO-$, $-NH-CO-O-CH_2CH_2-CO-$, $-NH-CO-O-CH_2-CH(CH_3)-CO-$, $(-NH-CO-O-CH_2)_2CH-O-CH_2CH_2CH_2-CO-$, $-O-CH_2-CH_2-NH-CO-O-CH_2CH_2-CO-$, and $(-O-CH_2-CH_2-NH-CO-O-CH_2)_2CH-O-CH_2CH_2CH_2-CO-$(wherein the -O- or -NH- present at the polymer side is shown for easier understanding), etc.

The coupling agent may be any one known in the art, in particular, known as one used for protein modification, and also known as a linker. Examples thereof include formaldehyde, glutaraldehyde, hexamethylene diisocyanate, hexamethylene diisothiocyanate, N,N'-polymethylene bisiodoacetamide, N,N'-ethylene bismaleimide, ethylene glycol bissuccinimidyl succinate, bisdiazobenzidine, 1-ethyl-3-(3-dimethylaminopropyl)-carbodiimide, succinimidyl 3-(2-pyridyldithio)propionate (SPDP), N-succinimidyl 4-(N-maleimidomethyl)cylcohexane-1-carboxylate (SMCC), N-sulfosuccinimidyl 4-(N-maleimidomethyl)cyclohexane-1-carboxylate, N-succinimidyl (4-iodoacetyl)aminobenzoate, N-succinimidyl 4-(1-maleimidophenyl)butylate, N-(ε-maleimidocaproyloxy)succinic acid imide (EMCS), iminothioran, S-acetylmercapto succinic acid anhydride, methyl-3-(4'-dithiopyridyl)propionimidate, methyl-4-mercaptobutylylimidate, methyl-3-mercapto-propionimidate, N-succinimidyl-S-acetylmercaptoacetate, etc.

The PEGylating reagent or PEGylation reagent may be selected from various types of those known in the art. The PEGylation reagents as can be conveniently used are those commercially available, for example, those available from NEKTAR, San Carlos, CA, USA. Examples of the commercially available PEG reagent include mPEG-succinimidyl propionate (mPEG-SPA) and branched PEG N-hydroxysuccinimide (mPEG2-NHS), but are not limited thereto. Some chemical formulae of typical PEG reagents are shown below:

[Chemical Formula 1]

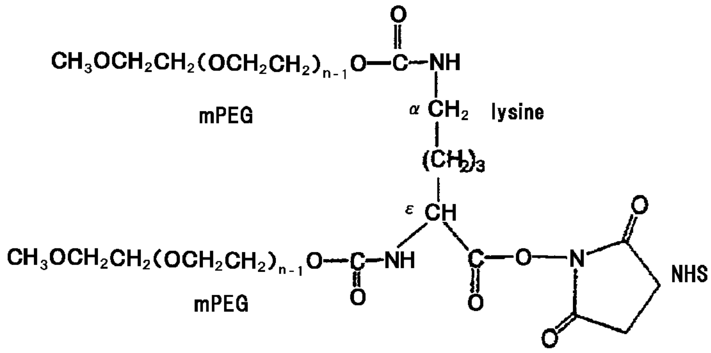

[Chemical Formula 2]

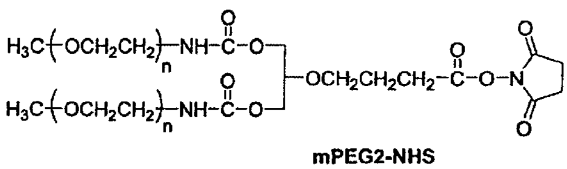

[Chemical Formula 3]

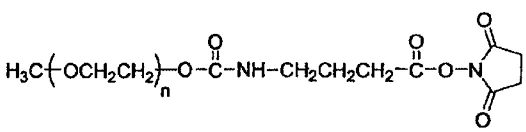

[Chemical Formula 4]

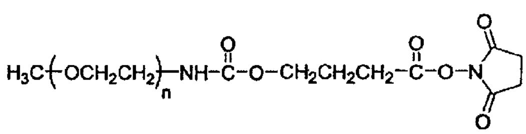

[Chemical Formula 5]

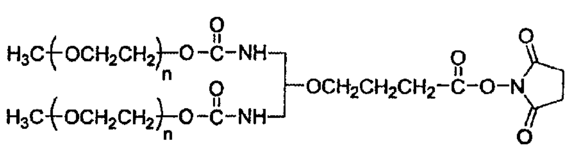

[Chemical Formula 6]

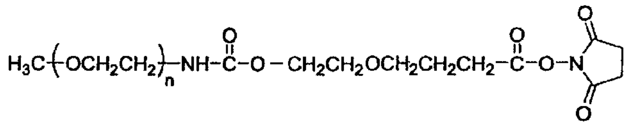

[Chemical Formula 7]

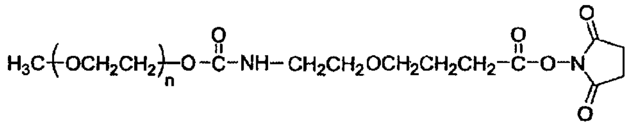

[Chemical Formula 8]

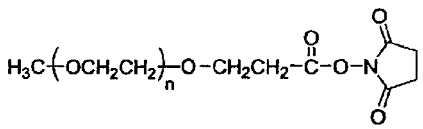

[Chemical Formula 9]

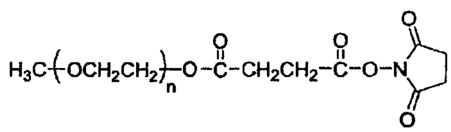

[Chemical Formula 10]

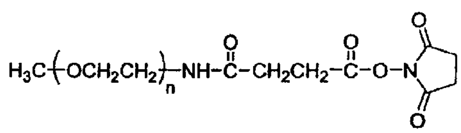

[Chemical Formula 11]

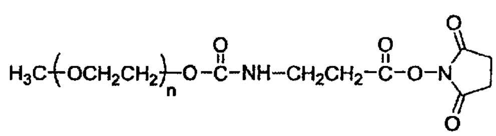

The binding (chemical attachment) of a polymer to galectin 9 (or modified galectin 9 proteins), i.e., the reaction of forming a conjugate, is referred to as conjugation or conjugating (or conjugation reaction). In a case that the polymer is PEG, this conjugation is referred as PEGylation or PEGylation reaction. In general, this reaction can be conducted in an aqueous solvent, but can be also conducted in an aqueous environment under conditions wherein conjugation-related

molecule species are adhered to carriers. The carrier as used herein can be suitably selected from those that are used in isolation/purification of protein, for example, selected from carries for affinity chromatography. Typical examples of the carrier are those having a ligand such as a sugar chain, for example, a carrier such as lactose-agarose in which β-galactoside is immobilized as a ligand to agarose.

It is preferable to take care that the conjugation reaction of the present invention is conducted so as to avoid modification of the amino group present on a lysine residue at a position important for the bioactivity of galectin 9. Also, it may be preferable, in some cases, to take care that the reaction is conducted so as to avoid modification of the amino group present on a lysine residue at a position important for the physiological activity of galectin 9. The present invention may encompass such care.

There is a possibility that the conjugation in a modified galectin 9 protein (which refers to stabilized human galectin 9 herein and, thereby, may be called as "stabilized galectin 9", and typical examples are those disclosed in WO 05/093064 (publication date: Oct. 6, 2005), and specific examples are those having an amino acid sequence of SEQ ID NO: 2 in Sequence Listing of WO 05/093064 (publication date: Oct. 6, 2005) and abbreviated to G9NC(null) herein) will occur in six lysine residues ($Lys^{88}$, $Lys^{95}$, $Lys^{111}$, $Lys^{180}$, $Lys^{238}$, and $Lys^{259}$ in the amino acid sequence of SEQ ID NO: 2 in Sequence Listing of WO 05/093064 (publication date: Oct. 6, 2005)) and in the N-terminal amino group thereof. Similarly, there is a possibility that the conjugation in M-type galectin 9 (Gal9M) will occur in six lysine residues ($Lys^{88}$, $Lys^{95}$, $Lys^{111}$, $Lys^{207}$, $Lys^{265}$, and $Lys^{286}$ in the amino acid sequence of SEQ ID NO: 2 in Sequence Listing of WO 02/037114 (publication date: May 10, 2002)) and in the N-terminal amino group thereof. Again similarly, there is a possibility that the conjugation in S-type galectin 9 (Gal9S) will occur in six lysine residues ($Lys^{88}$, $Lys^{95}$, $Lys^{111}$, $Lys^{195}$, $Lys^{253}$, and $Lys^{274}$ in the amino acid sequence of SEQ ID NO: 3 in Sequence Listing of WO 02/037114 (publication date: May 10, 2002)) and in the N-terminal amino group thereof. Further, there is a possibility that the conjugation in L-type galectin 9 (Gal9L) will occur in seven lysine residues ($Lys^{88}$, $Lys^{95}$, $Lys^{111}$, $Lys^{180}$, $Lys^{239}$, $Lys^{297}$, and $Lys^{318}$ in the amino acid sequence of SEQ ID NO: 1 in Sequence Listing of WO 02/037114 (publication date: May 10, 2002)) and in the N-terminal amino group thereof. Therefore, it is possible to obtain, for example, a conjugate molecule species in which a PEG molecule is bound to one of the aforementioned lysine residues (this molecular species may include positional isomers thereof), a conjugate molecule species in which PEG molecules are bound to two of the aforementioned lysine residues (this molecular species may include positional isomers thereof), a conjugate molecule species in which PEG molecules are bound to three of the aforementioned lysine residues (this molecular species may include positional isomers thereof), a conjugate molecule species in which PEG molecules are bound to four of the aforementioned lysine residues (this molecular species may include positional isomers thereof), and other conjugate molecule species. Preferred is a conjugate molecule species in which a PEG molecule is bound to one of the aforementioned lysine residues or a conjugate molecule species in which PEG molecules are bound to two of the aforementioned lysine residues.

For the conjugation, the amount of an activated polymer (such as a PEGylating reagent) used herein is not particularly limited to, with respect to the amount of galectin 9 used, but may be selected in the range that a desired modified product, i.e., a desired conjugate, can be obtained. For example, typically, the molar quantity of a PEGylating reagent can be in a range of from slightly lower to slightly higher than that of galectin 9. In some cases, the PEGylating reagent can be used in a molar quantity range of from slightly lower to excessive to that of galectin 9, i.e., about 0.8 to 8 times or about 1 to 8 times that of galectin 9. In some other cases, the PEGylating reagent can be used in an excessive molar quantity range of about 1.25 to 7 times or about 1.75 to 6 times that of galectin 9. The reaction temperature is not particularly limited to, but is generally ranging from 0 to 50˚C and preferably from about 4 to 40˚C. Typically, the reaction can be carried out at about 4˚C or room temperature. Also, the reaction can be carried out at 15 to 25˚C. The reaction time can be relatively short, for example, from 30 minutes to 3 hours or from about 1 to 2 hours in some cases, but is not limited thereto. The reaction can be terminated after a reaction for a predetermined period of time, which is preferred in some cases. The pH of the aqueous solvent in this reaction is ranging from about 7.0 to about 9.0 and preferably from about 7.5 to about 8.5. The reaction is preferably conducted in a buffer solution. The buffer solution may contain one or more members selected from phosphate ion, chlorine ion, bicarbonate ion, carbonate ion, and the like. For example, an aqueous solution containing one or more salts selected from NaCl, KCl, $Na_3PO_4$, $K_3PO_4$, $Na_2HPO_4$, $K_2HPO_4$, $(NH_4)_2HPO_4$, $NaH_2PO_4$, $KH_2PO_4$, $Na_2CO_3$, $K_2CO_3$, $NaHCO_3$, $KHCO_3$, $NH_4Cl$, $(NH_4)_2CO_3$, and the like is preferably used. Preferable buffer systems are, for example, those utilizing $Na_2HPO_4$-$NaH_2PO_4$ systems. Also, after the completion of the reaction, the buffer system can be changed into another buffer system. Coexisting ions can be changed by dialysis, gel filtration, and/or ion-exchange chromatography and further by affinity chromatography.

In the conjugation, the resulting reaction product solution may be obtained as a heterogeneous mixture of galectin 9 molecules containing polymer chains on different sites of the galectin 9 molecules. A solution containing these conjugates is useful even if it is used directly, but may be preferably subjected to isolation/purification based on molecular weights or difference in the positions of polymer chains on the galectin 9 molecules. The galectin 9-polymer conjugate in a reaction mixture obtained by the conjugation can be characterized by cation-exchange chromatography. For example, it may encompass identification of conjugates, such as conjugates having a polymer chain each at one position on a galectin 9 molecule, conjugates having a polymer chain each at a position different from that of the previous conjugate,

conjugates having polymer chains at two positions on a galectin 9 molecule, and conjugates having two polymer chains at different positions from those of the previous conjugate, like in positional isomers. In the present invention, a mixture containing galectin 9-polymer conjugate positional isomers and/or a mixture of galectin 9-polymer conjugate molecule species having different numbers of polymer chains on a galectin 9 molecule can be used. A conjugate-containing solution may be in a state of a mixture containing at least two positional isomers, a mixture containing three galectin 9-polymer conjugate positional isomers in some cases, or further a mixture containing four or more galectin 9-polymer conjugate positional isomers.

The target product contained in a reaction product solution obtained by the conjugation can be purified with suitable combinations of publicly well known isolation/purification methods. The target product can be purified, for example, by salting-out such as ammonium sulfate precipitation; gel filtration using Sephadex™ or the like; ion-exchange chromatography using a carrier having, for example, a diethylaminoethyl group, a carboxymethyl group, or an $-SO_3^-$ group; hydrophobic chromatography using a carrier having a hydrophobic group, such as a butyl group, an octyl group, or a phenyl group; dye-gel chromatography; electrophoresis; dialysis; ultrafiltration; affinity chromatography; or high-performance liquid chromatography. Preferably, the isolation/purification can be conducted by polyacrylamide gel electrophoresis and affinity chromatography utilizing an immobilized ligand. The ligands includes, for example, antibodies, including monoclonal antibodies, showing specific recognition, fragments thereof, lectin, saccharides, and one member of a binding pair. The isolation/purification as can be used herein may be, for example, immunoaffinity chromatography or chromatography using a carrier such as agarose immobilizing β-galactoside as a ligand, including gelatin-agarose affinity chromatography, heparin-agarose chromatography, and lactose-agarose chromatography. A desired fraction can be preferably obtained by ion-exchange chromatography using a strong-acid cation exchange resin or a strong-acid cation exchange gel. In some cases, the application of gel filtration chromatography, which enables separation based on molecular size, is effective, and also the application of anion-exchange chromatography is effective in some other cases. Concentration gradients can be utilized in the elution of a target conjugate from a carrier. The elution with an aqueous solvent is preferred, and buffer solutions can be advantageously used. The buffer solution may contain one or more members selected from phosphate ion, chlorine ion, bicarbonate ion, carbonate ion, and the like, and, for example, an aqueous solution containing one or more salts selected from NaCl, KCl, $Na_3PO_4$, $K_3PO_4$, $Na_2HPO_4$, $K_2HPO_4$, $(NH_4)_2HPO_4$, $NaH_2PO_4$, $KH_2PO_4$, $Na_2CO_3$, $K_2CO_3$, $NaHCO_3$, $KHCO_3$, $NH_4Cl$, $(NH_4)_2CO_3$, and the like is preferably used. Preferable buffer systems are, for example, those utilizing a $Na_2HPO_4$-$NaH_2PO_4$ system. The pH of the aqueous solvent in this fractionation is not particularly limited to but is usually ranging from about 5.0 to about 10.0, preferably from about 6.0 to about 9.0, and more preferably from about 7.0 to about 8.0, as long as a product is not adversely affected. The processing temperature for the elution is not particularly limited to but can be usually ranging from 0 to 37˚C, preferably from about 4 to 30˚C. Elution can be conducted at about 4˚C or around room temperature in typical cases and can be conducted at about 4 to 25˚C.

In typical cases, a column packed with a cation-exchange gel is equilibrated with a aqueous buffer solution having the same pH and salt concentration as those of a conjugated galectin 9 solution, by a conventional technique. Then, the solution containing the conjugate is applied to the column for adsorption. According to need, a buffer solution having the same pH and salt concentration as those of the conjugated galectin 9 solution is optionally applied to the column for eluting unadsorbed components. Then, an elution buffer is applied to the column while gradually increasing the salt concentration to form a concentration gradient, and the eluate is collected in fractions. The galectin 9-polymer conjugate in each fraction can be further isolated/purified so as to have a substantially uniform molecular weight and degree of substitution. The galectin 9-polymer conjugate fraction may preferably contain galectin 9 conjugate molecules having one to six polymer chains per one molecule of galectin 9 (for example, in a case using mPEG2-NHS as a PEGylating reagent, when the mPEG2-NHS is bound to one of amino groups present at amino acid residues on galectin 9 (one substitution-binding), it means an incorporation of two mPEG, i.e., two polymer chains). In some cases, the fraction may contain galectin 9 conjugate molecules having one to four polymer chains per one molecule of galectin 9, may contain galectin 9 conjugate molecules having one to two polymer chains per one molecule of galectin 9, or may contain galectin 9 conjugate molecules having one polymer chain per one molecule of galectin 9.

The galectin 9-polymer conjugates obtained according to the present invention can be advantageously employed as pharmaceutically-active components. In a case of using the conjugates as the active components, the galectin 9-polymer conjugate may be isolated/purified into a single molecular state, or may be a fraction containing a galectin 9-polymer conjugate having a substantially uniform molecular weight and degree of substitution, or may be in a state of a mixture containing a plurality of galectin 9-polymer conjugate species, said mixture being recognized to be therapeutically homogeneous. These galectin 9-polymer conjugates can be suitably selected and used by taking the object and the functional effect into consideration.

Where the active components of the present invention are used as pharmaceutical drugs, they can be generally administered alone or in the form of a pharmaceutical composition or preparation in admixture with any of various pharmaceutically acceptable aids. Preferably, it may be administered in the form of a convenient pharmaceutical composition or formulation for oral, topical, parenteral application, and the like. Any of dosage forms (including those for inhalation

and rectal administration) may be selected depending on purpose. Each active component of the present invention may be used in the form of a mixture thereof. Examples of said mixture as can be used herein are a mixture of a galectin 9-polymer conjugate and native galectin 9, a mixture of a galectin 9-polymer conjugate and a modified galectin 9 protein, a mixture of galectin 9-polymer conjugate and a galectin 9-inducing factor, a mixture of a galectin 9-polymer conjugate, galectin 9, and a galectin 9-inducing factor, or a mixture of native galectin 9, a galectin 9-polymer conjugate, and a modified galectin 9 protein, etc.

Furthermore, the active components of the present invention can be used in combination with any of various drugs, including, for example, anti-tumor drugs (antineoplastic drugs), antibiotics, tumor metastasis-inhibitors, thrombogenesis inhibitors, therapeutic drugs for joint destruction, analgesics, anti-inflammatory drugs, anti-allergy drugs, immunomodulators (or immunoregulators) and/or immunosuppressants, which can be employed as not being restricted to particular species as long as they serve effectively or advantageously. For instance, they can be optionally selected from those known in the art.

The drug can be preferably administered to mammals including human parenterally (e.g., intracellularly, intratissularly, intravenously, intramuscularly, subcutaneously, intracutaneously, intraperitoneally, by intra-thoracic routes, intraspinally, by instillation, enterally, per rectum, by instillation into the ear, eye, nose by swabbing or application on the teeth, skin, or mucosa, etc.). Specific dosage forms of the pharmaceutical preparations and formulations include solutions, suspensions, semi-solids, particulates, shaped preparations, and extractives, etc. Examples of the dosage forms are microcapsules, implants, powders, pulvis, granules, fine granules, injections, liquids and solutions, elixirs, emulsions, irrigations, aqueous liquids, creams, suspensions, aerosols, sprays, inhalations, air sprays, dermatologic waters, eye drops, nose drops, ear drops, liniments, infusion solutions, powders for injection solutions or the like, lyophilized preparations, and conditioned gels, etc.

The pharmaceutical compositions can be formulated according to conventional techniques. For example, according to need, a unit dose form that is required for generally approved pharmaceutical practices can be given by optionally admixing the conjugate of the present invention or the like with a single member, or suitably a combination thereof, selected from the group consisting of physiologically acceptable carriers, pharmaceutically acceptable carriers, adjuvants, excipients, vehicles, diluents, flavoring agents, essences, sweeteners, expanders, antiseptics, stabilizers, binders, pH regulators, buffers, detergents (surfactants), bases, solvents, fillers, extenders, dissolution adjuvants, solubilizers, isotonizing agents, emulsifiers, suspending agents, dispersing agents, thickeners, gelling agents, stiffening agents, absorbents, adhesives, flexing agents, plasticizers, collapsing agents, emitting agents, preservatives, anti-oxidants, shading agents, humectants, mitigatives, antistatic agents, analgesic agents, etc.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, an aqueous dextrose solution, other related saccharide solutions, ethanol, glycols such as propylene glycol and polyethylene glycol, etc. For the preparation of injections, the active component is usually admixed with any of carriers, such as distilled water, Ringer's solution, or physiological saline, suitable dispersing agents, moistening agents, suspending agents, and other materials to form injectable formulations including solutions, suspensions, emulsions, etc, by known techniques in the art. Examples of aqueous solutions for injection include physiological saline and isotonic solutions containing glucose or other aids (e.g., D-sorbitol, D-mannitol, sodium chloride, etc.), where they may be used in combination with a suitable pharmaceutically acceptable auxiliary solubilizer such as alcohol (e.g., ethanol, etc.), polyalcohol (e.g., propylene glycol, polyethylene glycol, etc.), nonionic surfactant (e.g., Polysorbate 80™, HCO-50, etc.) and others. The injectable oily liquids may include sesame oil, soybean oil, and other materials, where they may be used in combination with benzyl benzoate, benzyl alcohol, or the like as a dissolution adjuvant. Furthermore, buffers (e.g., phosphate buffer, sodium acetate buffer, etc.), or agents for osmoregulation, analgesic agents (e.g., benzalkonium chloride, procaine hydrochloride, etc.), stabilizers (e.g., human serum albumin, polyethylene glycol, etc.), preservatives (e.g., benzyl alcohol, phenol, etc.), anti-oxidants such as ascorbic acid, absorbefacients, etc. may be blended. The prepared injection is usually filled in a suitable ampule.

For parenteral administration, solution or suspension unit dosage forms are prepared in pharmaceutically acceptable sterile fluids, such as water, ethanol, and oils, in admixture with or without detergent or other pharmaceutically acceptable aids. The oily vehicle and solvent used in the parenteral formulation may include native, synthetic, semi-synthetic mono-, di-, or tri-glycerides; native, semi-synthetic, or synthetic fats and oils; and fatty acids. Examples of such oily vehicles and solvents are plant oils such as peanut oil, corn oil, soybean oil, and sesame oil. For example, this injection can be usually prepared so as to form unit doses each containing from about 0.1 to 10 wt% of the compound of the present invention.

The active components of the present invention can be expected as pharmaceutical drugs including an anti-tumor agent (anticancer agent), an anti-allergy agent, an immunosuppressive agent, an anti-autoimmune disease agent, an anti-allergy agent, an anti-inflammatory agent, or an adrenocortical steroid hormone substitute. The active component of the present invention has an activity of inducing apoptosis in activated T cells and is useful for an application using

this activity. The active component of the present invention is excellent so as to achieve advantageous characteristics, such as a reduction or elimination in adverse side effects (including inhibition of hemagglutination), an increase in solubility, improvements in pharmacodynamics in vivo and pharmacokinetics in vivo (for example, prolongation of action potential duration, such as prolongation of blood circulation half-life), and preferable in vivo biodistribution, as an immunoregulatory agent or an anti-tumor agent, for example, an anti-tumor agent for a tumor selected from the group consisting of cancers and sarcomas including a brain tumor (glioblastoma multiforme), spinal cord tumor, maxillary sinus cancer, pancreatic adenocarcinoma, gingiva cancer, tangue cancer, lip cancer, nasopharyngeal carcinoma, mespharyngeal carcinoma, hypopharyngeal carcinoma, laryngeal cancer, thyroid cancer, parathyroid cancer, lung cancer, pleural tumor, cancerous peritonitis, cancerous pleurisy, esophagus cancer, gastric cancer, colon cancer, bile duct cancer, gallbladder cancer, pancreatic cancer, liver cancer, renal cancer, bladder cancer, prostatic cancer, penile cancer, a testicular tumor, adrenal cancer, cervical cancer, uterine cancer, vaginal cancer, vulval cancer, ovarian cancer, chorioepithelioma, malignant bone tumor, soft tissue sarcoma, breast cancer, skin cancer, malignant melanoma, basal cell tumor, leukemia, myelofibrosis with myeloid metaplasia, malignant lymphoma, Hodikin's disease, plasmacytoma, and glioma; and a drug for the following disorder, disease, or pathological symptom:

(A) an inflammatory disease selected from the group consisting of various types of acute and chronic inflammation occurring in each organ, allergic and autoimmune inflammation, and infectious diseases;
(B) an acute or chronic disease selected from the group consisting of lung diseases, including bronchitis, bronchopneumonia, interstitial pneumonitis, pneumonia, bronchiolitis, and acute mediastinitis; diseases of organs other than lung, including epicarditis, endocarditis, myocarditis, stomatitis, angular cheilitis, amygdalitis, adenoiditis, laryngitis, esophagitis, peritonitis, acute gastritis, chronic gastritis, acute enteritis, apendicitis, ischemic colitis, colitis midicamentosa, and proctitis; and inflammation, including hepatitis A, hepatitis B, hepatitis C, fulminant hepatitis, acute and chronic hepatitis, hepatic cirrhosis, cholecystitis, acute pancreatitis, chronic pancreatitis, acute and chronic nephritis, membranous nephritis, glomerulonephritis, IgA nephritis, various types of cystitis, encephalomyelitis, mastitis, dermatitis, superficial keratitis, xerotic keratitis, tympanitis, rhinitis, paranasal sinusitis, nasal polyp, gingivitis, periodontitis, and pericoronitis;
(C) a symptom selected from the group consisting of nervous inflammation (for example, neurotic gastritis and neurotic cystitis) and pain caused by cancer or inflammation;
(D) an allergic inflammatory disease selected from the group consisting of systemic anaphylaxis, bronchial asthma, hypersensitive pneumonitis, pollinosis, allergic rhinitis, allergic conjunctivitis, allergic diseases caused by immune complexes, and angioneurotic edema;
(E) autoimmune inflammation (autoimmune disease) selected from the group consisting of sytemic disorders (e.g., rheumatoid arthritis, systemic erythematosus, polyarteritis nodosa, scleroderma, polymyositis/dermatomyositis, Sjogren's syndrome, and Behoet's syndrome), neural system (e.g., multiple sclerosis, myasthenia gravis, HAM (HTLV-1 myelopathy), and amyotrophic lateral sclerosis), endocrine system (e.g., hyperthyroidism, chronic thyroiditis, and type 1 diabetes mellitus), blood (e.g., idiopathic thrombocytopenic purpura, autoimmune hemolytic anemia, and aplastic anemia), respiratory system (e.g., sarcoidosis and pulmonary fibrosis), gastrointestinal system (e.g., ulcerative colitis and Crohn's disease), liver (e.g., autoimmune hepatitis, primary biliary cirrhosis, primary sclerosing cholangitis, and autoimmune cholangitis), nephro-urinary system (e.g., antineutrophil cytoplasmic antibody-associated nephritis, angiitis, Goodpasture's syndrome, and antiglomerular basement-membrane antibody disease);
(F) an infectious disease that is caused by that pathogenic organisms damage cells, tissues, and an organ of a living body or is caused by pathogenic organisms that cause an infectious disease in a human, where the pathogenic organisms are selected from the group consisting of 1) bacteria (including spirochete, chlamydia, and rickettsia), 2) viruses, 3) fungi, 4) plants (algae), 5) protozoa, 6) parasites (trematodes, cestodes, and nematodes), and 7) arthropods, and the infection is selected from the group consisting of bacterial infectious diseases (e.g., cholera, pest, and Escherichia coli infection), spirochaetosis (e.g., leptospirosis), chlamydia infectious diseases (e.g., psittacosis), rickettsial infectious diseases (e.g., eruptive fever and tetanus), viral infectious diseases (e.g., herpes zoster, viral hemorrhagic fever, and hydrophobia), fungous diseases (e.g., candidiasis, cryptococcosis, and aspergillosis), protozoan diseases (e.g., amebic dysentery, malaria, and toxoplasmosis), parasites (e.g., trematode infection and nematode infection), mycoplasma infectious diseases (e.g., mycoplasma pneumonia), and mycobacteria infectious diseases (e.g., tuberculosis and atypical mycobacteriosis);
(G) a disease in dermatology field, selected from i) cutaneous infectious diseases, dermatitis including allergic inflammation and autoimmune inflammation, and cutaneous disorders having characteristic inflammation, such as psoriasis, hydroa, pustuliform, keratinization, or keratinization abnormality, and ii) beauty dermatology-associated disorders or aging in dermatology field (including aging) relating to a) melanin metabolism control (whitening), b) hair growth (hair growth) control, and c) collagen production control;
(H) a lifestyle-related disease selected from the group consisting of hyperlipemia, arteriosclerosis, hypertension, and diabetes mellitus;

(I) pathosis in maintaining normal bacterial flora;
(J) a disease selected from the group consisting of amyloidosis, Alzheimer's disease, osteoporosis, and bone fracture;
(K) inflammatory reaction in the cranial nerve region, for example, inflammation accompanied by development of an ischemic disease, such as brain infarction or myocardial infarction, or schizophrenia;
(L) gout;
(M) osteoporosis; or
(N) interstitial pneumonia.
The terms used in the specification and drawings are in accordance with UPAC-IUB Commission on Biochemical Nomenclature or are based on the meanings of terms commonly used in the art.
DTT: thiothreitol
PMSF: phenylmethylsulfonyl fluoride
PBS: phosphate-buffered aqueous salty solution or phosphate-buffered saline

Details of the present invention are described by the following examples but such examples are provided only for illustrative purposes, and for referential embodiments of the present invention. These examples have been described herein for the purpose of illustrating specific embodiments of the present invention but should not be construed as in any sense limiting the scope of the invention disclosed herein. It should be understood in the present invention that various embodiments can be made or executed within the spirit, scope and concept disclosed herein. All the examples were carried out or can be carried out, unless otherwise disclosed herein specifically, by standard techniques which are well known and conventional to those skilled in the art. The terms "5K SE2, 10K SE2, 20K SE2, and 40K SE2" refer to stabilized galectin 9 (modified galectin 9 protein or Gal-9 mutein, G9NC(null)) conjugated with PEGylating reagents having molecular weights of 5000, 10000, 20000, and 40000, respectively.

Reference Example 1

Galectin 9 used in the following Examples was stabilized galectin 9, prepared according to Example 1 of WO 05/093064 (publication date: Oct. 6, 2005), i.e., G9NC(null) (polypeptide encoded by a nucleotide sequence of SEQ ID NO: 1 and having an amino acid sequence of SEQ ID NO: 2 in WO 05/093064 (publication date: Oct. 6, 2005)).
The stabilized galectin 9 (modified galectin 9 protein), i.e., G9NC(null), can be also prepared as follows:

The expression of stabilized galectin 9 (G9NC(null)) was induced by culturing Escherichia coli (BL21 (DE3)) electroporatically transformed with by pET-G9NC(null) in a 2 x YT medium containing 2% (w/v) glucose and 100 μg/mL ampicillin and adding thereto isopropyl-β-D-thiogalactopyranoside to make a final concentration of 0.15 mM when the absorbance at 600 nm reached 0.7. After incubation at 20˚C for 22.5 hours, the bacterial cells were centrifugally collected and were stored at -80˚C until purification. The lyophilized bacterial cells were well suspended in 10 mM Tris-HCl (pH 7.5), 0.5 M NaCl, 1 mM DTT, 1 mM PMSF, 10 mM $MgCl_2$, 25 μg/mL DNase, and 0.2 mg/mL egg-white lysozyme, and Triton X-100 was added thereto to make a final concentration of 1%. The mixture was sonicated for 18 minutes and was centrifuged at 18800 g for 75 minutes. The resulting supernatant was subjected to affinity chromatography using lactose-agarose for purifying the recombinant protein, and then the buffer was replaced with PBS by dialysis. Then, the precipitate was removed through a 0.22 μm sterilized filter, and endotoxin was removed by treatment with cellulofine ET-Clean L. Then, various bacteria and precipitates were further removed through a 0.22 μm sterilized filter to give a final G9NC(null) sample.

No contaminant bands were observed by the protein polyacrylamide electrophoresis in the G9NC(null) thus prepared, and the amount of endotoxin is less than 0.5 EU/mL. The G9NC(null) is stable to freezing-thawing and stably maintains the bioactivity for a half year or more, even if stored at 4˚C.
Galectin 9 may include recombinant form proteins prepared by expressing native L-type galectin 9, M-type galectin 9, or S-type galectin 9 in host cells with "gene recombination technology ".

Example 1

Preparation of PEGylated stabilized galectin 9

a) A column was packed with 8 mL of a lactose-agarose gel bound with stabilized galectin 9 (G9NC(null)) that was obtained in a cell supernatant of Escherichia coli used as a transformed host cell (the amount of stabilized galectin

9 bound to the gel was about 15 mg per 1 mL of the gel). The gel was washed with a 3-fold gel volume (24 mL) of 50 mM $Na_2HPO_4$-$NaH_2PO_4$ (pH 8.0) and 150 mM NaCl. The washed gel was used as a recombinant galectin 9 source.

b) A PEGylating reagent was dissolved in 24 mL of 25 mM $Na_2HPO_4$-$NaH_2PO_4$ (pH 8.0), 75 mM NaCl, and 100 mM lactose, and the resulting solution was immediately applied to the washed gel prepared in the above a). Four types of PEGylating reagents shown below were used.

[Table 1]

Table 1

| PEGylation Reagent | Molecular Weight | Seller / Item No. | Applied Dose per 8 mL (Gel) |
|---|---|---|---|
| mPEG -SPA | 5,000 | NEKTAR/2M4M0H01 | 90 mg |
| mPEG2-NHS | 10,000 | NEKTAR/2Z3Y0L01 | 180 mg |
| mPEG2-NHS | 20,000 | NEKTAR/2Z3Y0P01 | 360 mg |
| mPEG2-NHS | 40,000 | NEKTAR/2Z3Y0T01 | 720 mg |

mPEG-SPA: methoxy poly(ethylene glycol) succinimidyl propionate
c) A mixture of each of the above-mentioned PEGylating reagents and the recombinant galectin 9 source gel was put into a Conical tube (Nippon Becton Dickinson Co., Japan) and was stirred (with a rotator) at room temperature (20 to 22°C) for one hour.

d) The resulting PEGylating reagent-gel mixture was packed in a column and the effluent (E1) was collected. Then, the gel was washed with 12 mL of TBS (20 mM Tris-HCl, pH 7.5, 150 mM NaCl) and 200 mM lactose, and the effluent (E2) was collected.

e) E1 and E2 were mixed, and the mixture was dialyzed against 1 L of 20 mM MES-NaOH (pH 6.0) (first time: 4 hours, second time: 12 hours, third time: 6 hours). MES means 2-morpholinoethanesulfonic acid.

f) The dialyzed solution was centrifuged (15000 g, 20 minutes). The supernatant was collected and filtered through a 0.2 μm filter, and the filtrate was collected.

Ion-exchange chromatographic purification of PEGylated protein

The chromatographic conditions are as follows:

a) Ion-exchange column: RESOURCE S (6 mL) (GE Healthcare, 17-1180-01) was used, and AKTAprime (GE Healthcare) was used as a purification apparatus. A half amount of the sample prepared in the above-step d) was applied to the column dividedly in several times (with a 5 mL sample loop).

b) The column was washed with 25 mL of Buffer A (20 mM MES-NaOH (pH 6.0), 20 mM lactose) and then eluted with a 0 to 30% gradient in 300 mL of Buffer B (20 mM MES-NaOH (pH 6.0), 20 mM lactose, 500 mM NaCl) at a flow rate of 5 mL/min in a fraction amount of 6 mL/fraction.

c) A fraction (SE1; refer to FIG. 1) containing mainly a modified molecule in which a plurality of PEG molecules were induced into one stabilized galectin 9 molecule and a fraction (SE2) containing mainly a modified molecule in which one PEG molecule was induced into one stabilized galectin 9 molecule were each collected and were each concentrated using a single hollow-fiber concentrator.

d) The concentrated samples were dialyzed against 1 L of PBS (first time for 4 hours; second time for 12 hours; third time for 6 hours).

e) The dialyzed solution was centrifuged (15000 g, 20 minutes). The supernatant was collected and was sterilized by filtration through a sterilized filter to give a purified PEGylated stabilized galectin 9 sample.

The protein elution pattern of PEGylated stabilized galectin 9 (PEGylated G9NC(null)) by ion-exchange chromatography using RESOURCE S column (GE Healthcare Bioscience Corp.) and the SDS-polyacrylamide gel electrophoretic results of each fraction were similar to those shown in FIG. 1. FIG. 1 shows data obtained for stabilized galectin 9 PEGylated with mPEG2-NHS having a molecular weight of 20000 (mPEG2-NHS (20K) PEGylated G9NC(null)) (the solid line shows the absorbance at 280 nm, and the dotted line shows the electric conductivity).
The stabilized galectin 9 PEGylated with mPEG2-NHS having a molecular weight of 20000 was roughly separated into four fractions (SE1 to SE4) (Other PEGylated reagents also showed substantially the same results). The results of SDS-PAGE confirmed that each fraction contained the following molecular species:

SE1: mainly containing molecular species having the PEG molecule at two or more sites,
SE2: mainly containing molecular species having the PEG molecule at one site, and
SE3 & SE4: mainly containing non-PEGylated molecular species.

The actual mobility of the PEGylated stabilized galectin 9 was less than that expected from the molecular weight (the PEGylated G9NC(null) migrated to the site at which migrated molecules with a molecular weight higher than a calculated molecular weight were supposed to be positioned). This is thought that SDS is hardly bound to the PEG portion.
Since stabilized galectin 9 (G9NC(null) includes six lysine residues ($Lys^{88}$, $Lys^{95}$, $Lys^{111}$, $Lys^{180}$, $Lys^{238}$, and $Lys^{259}$ in the amino acid sequence of SEQ ID NO: 2 in Sequence Listing of WO 05/093064 (publication date: Oct. 6, 2005)), there are seven sites, including the amino group at the N-terminal, having the possibility that the PEG molecule will bind thereto. SE1 and SE2 are suggested to be a mixture of molecular species that are different from each other in the sites to be PEGylated. SE3 and SE4 are suggested to have the possibility that molecules modified with impurities (e.g., small molecular weight modifying reagents) present in the PEGylating reagents would be contained.

Example 2

Bioactivity of PEGylated stabilized galectin 9

1. Cell proliferation-inhibition assays

Cell proliferation-inhibiting activity (cytotoxicity) against PC-3 (human prostate cancer cell line) and MOLT-4 (human T-cell leukemia line) was investigated. The cytotoxicity against PC-3 was evaluated by a test measuring absorbance after addition of WST-8 (1-Methoxy PMS). The cytotoxicity against MOLT-4 was evaluated by a test measuring absorbance after addition of WST-1 (4-[3-(4-iodophenyl)-2-(4-nitrophenyl)-2H-5-tetrazolio]-1,3-benzene disulfonate).

1) Cytotoxicity against PC-3
Cells suspended in Dulbecco's modified Eagle's medium (Gibco BRL) and 10% FBS (Thermo Trace Ltd.) were seeded in a 96-well plate (FALCON) at 3 x $10^3$ cells/well (90 μL) and cultured at 37˚C for 48 hours in 5% $CO_2$, and 10 μL of PEGylated stabilized galectin 9 or stabilized galectin 9 was added to each well to make a final concentration of 0.1 μM, 0.3 μM, 1μ M, or 2 μM, respectively. The mixture was cultured at 37˚C for 24 hours (control: PBS). After the culturing, 10 μL of a WST-8 reagent (Wako, 343-07623) was added to each well, and the mixture was cultured at 37˚C for 2 hours in 5% $CO_2$. Then, absorbance at 450 to 600 nm was measured with a plate reader. PEGylated stabilized galectin 9 and stabilized galectin 9 were applied to the measurement at the same time, and activities were evaluated by comparing the results.

2. Assay results

The test results are shown in FIGs. 2 to 5. FIGs. 2 to 5 show the results of proliferation-inhibiting activity test of purified PEGylated stabilized galectin 9 samples against PC-3 cells. FIG. 2 shows the results of investigated activities of stabilized galectin 9 PEGylated with mPEG-SPA having a molecular weight of 5000 (G9Null PEG 5K SE2) or with mPEG2-NHS having a molecular weight of 10000 (G9Null PEG 10K SE2), i.e., G9Null-PEG-5K-SE2 (G9NC(null)-PEG-5K-SE2) or G9Null-PEG-10K-SE2 (G9NC(null)-PEG-10K-SE2). G9Null PEG 5K SE2 (hereinafter, 5K SE2) showed approximately the same activity (specific activity) as that of stabilized galectin 9, and G9Null PEG 10K SE2 (hereinafter, 10K SE2) showed slightly higher activity (specific activity) than that of stabilized galectin 9. Since this activity test investigates inhibitory activity against cell proliferation, there is a possibility that measured activity is apparently higher than the actual activity when the sample contains a material cytotoxic to cultured cells. However, the activities of 5K SE2 and 10K SE2 were inhibited by lactose, as in stabilized galectin 9, and, consequently, the possibility that this activity is caused by contamination of non-specific toxic materials derived from a PEGylating reagent was denied.
FIG. 3 shows the results of investigated activities of stabilized galectin 9 PEGylated with mPEG2-NHS having a molecular

weight of 20000 (G9Null PEG 20K SE2) or with mPEG2-NHS having a molecular weight of 40000 (G9Null PEG 40K SE2), i.e., G9Null-PEG-20K-SE2 (G9NC(null)-PEG-20K-SE2) or G9Null-PEG-40K-SE2 (G9NC(null)-PEG-40K-SE2). Both G9Null PEG 20 SE2 (hereinafter, 20K SE2) and G9Null PEG 40K SE2 (hereinafter, 40K SE2) showed higher activity than that of stabilized galectin 9. The results of inhibition by lactose were the same as those shown in FIG. 2. FIG. 4 shows a comparison between the SE1 fraction and the SE2 fraction (G9Null PEG 5K and G9Null PEG 10K). In order to investigate the relationship between proliferation-inhibiting activity against PC-3 cells and the binding amount (binding number) of PEG molecules, the SE1 fraction and the SE2 fraction were compared. The activities of SE1 fractions were low for both 5K and 10K. From this, it is verified that the activity was decreased by the modification with a plurality of PEG molecules. There is a possibility that a difference in the modification site causes a difference in the activity when stabilized galectin 9 is modified with a single PEG molecule. FIG. 5 shows a comparison between the SE1 fraction and the SE2 fraction (G9Null PEG 20K and G9Null PEG 40K). In the cases of 20K and 40K, the results were the same as those of 5K and 10K.
For the proliferation-inhibiting activity against PC-3 cells, 5K SE2 exhibited approximately the same activity as that of stabilized galectin 9, and 10K SE2, 20K SE2, and 40K SE2 exhibited higher activities (in vitro) than that of stabilized galectin 9. Regarding MOLT-4, similar results were obtained.

Example 3

Bioactivity of PEGylated stabilized galectin 9

1. Hemagglutination assays

Hemagglutination activity against rabbit erythrocytes and human erythrocytes were investigated by a visual inspection test of evaluating hemagglutination reaction against erythrocytes. The stabilized galectin 9 and PEGylated stabilized galectin 9 were applied to the measurement at the same time, and the activity was evaluated by comparing the results.

1) Human hemagglutination assays

Human hemagglutination assay for evaluating hemagglutination activity was conducted according to the method of Nowak, et al. (Nowak, et al., Biochem. Biophys. Res. Commun., 68: 650-657 (1976)).
Stabilized galectin 9 or PEGylated stabilized galectin 9 (5K, 10K: 0.05 to 100 μg/mL, 20K, 40K: 0.1 to 100 μg/mL) was prepared as assay samples in a 96-well plate (FALCON). Bovine serum albumin was dispensed into each well to make a final concentration of 0.25% (w/v), and then citric acid- or EDTA-treated, type AB or O whole blood wherein the final concentration of contained erythrocytes was adjusted to 1% (v/v) was added to the well to make a total liquid volume of 100 μL. The resulting reaction mixture was incubated at room temperature for 1 hour, and the minimum concentration necessary for agglutination was determined by visual inspection.

2) Rabbit hemagglutination assays

a) Preparation of glutaraldehyde-fixed trypsin-treated rabbit erythrocytes
Rabbit blood (100 mL, purchased from Shimizu Laboratory Supplies Co., Ltd., Japan) was centrifuged at 2000 rpm for 15 minutes, and the supernatant was removed. The precipitate was washed with 0.15 M NaCl, and 0.1 M Na-phosphate/0.05 M NaCl was added thereto to adjust the erythrocyte concentration to 4%. Trypsin (final concentration: 1 mg/mL) (SIGMA) was added to the erythrocyte solution, and the mixture was incubated at 37˚C for 1 hour. After centrifugation at 2300 rpm for 15 minutes, the supernatant was removed. The precipitate was washed with 0.15 M NaCl, and glutaraldehyde (SIGMA) and PBS (pH 7.2) were added thereto to adjust the glutaraldehyde concentration to be 1% for fixation at room temperature for 1 hour. Then, after centrifugation, the supernatant was removed. After washing with 0.1 M glycine (WAKO)/PBS (pH 7.2) followed by centrifugation, the supernatant was removed. After washing with PBS (pH 7.2), the supernatant was removed. The resulting erythrocytes were stored as a 10% erythrocyte suspension in 0.05% $NaN_3$/PBS (pH 7.2) at 4˚C.

b) Assays
Hemagglutination assay for hemagglutination activity was conducted according to the method of Nowak, et al. (Biochem. Biophys. Res. Commun., 68: 650-657 (1976)). Stabilized galectin 9 or PEGylated stabilized galectin 9 (5K, 10K: 0.05 to 100 μg/mL, 20K, 40K: 0.1 to 100 μg/mL) was prepared as assay samples in a 96-well plate

(FALCON). Bovine serum albumin was dispensed into each well to make a final concentration of 0.25% (w/v), and the aforementioned glutaraldehyde-fixed trypsin-treated rabbit erythrocytes wherein the final concentration of contained erythrocytes was adjusted to 1% (v/v) was added to the each well to make a total liquid volume of 100 μL. The reaction mixture was incubated at room temperature for 1 hour, and the minimum concentration necessary for agglutination was determined by visual inspection.

2. Assay results

The assay results are shown in FIG. 6. In order to investigate effects of PEGylation on stabilized galectin 9 hemagglutination activity, the activity was measured using rabbit erythrocytes (erythrocytes that was treated with trypsin, and then fixed with glutaraldehyde), which were generally used in detection of lectin activity, and fresh human erythrocytes (used erythrocytes are types O and AB in consideration of carbohydrate antigens). The concentration of stabilized galectin 9 exhibited agglutination activity against rabbit erythrocytes was 12.5 to 25 nM (the minimum concentration exhibiting agglutination activity was used as an index for the activity). On the other hand, the PEGylated stabilized galectin 9 fractions, 10K SE2, 20K SE2, and 40K SE2, did not exhibit agglutination activity even at a concentration of 12.8 μM and were verified to be substantially inactive in this assay. 5K SE2 exhibited an activity with about a quarter (minimum agglutination concentration: 50 to 100 nM) of that of stabilized galectin 9. It is unclear why the agglutination activity of 5K SE2 was not detected at the higher concentration side (about 1.6 μM or more), but the same phenomenon was also observed in stabilized galectin 9. Possible reasons are that when the concentration of galectin is high, sugar chain ligands on erythrocyte membranes may be saturated with galectin and therefore cross-linking between erythrocytes is suspected not to be formed.
Also, in a case of fresh human erythrocytes, stabilized galectin 9 exhibited agglutination activity at concentrations of 100 to 200 nM, but 10K SE2, 20K SE2, and 40K SE2 did not exhibit agglutination activity even at a concentration of 12.8 μM.
Thus, 10K SE2, 20K SE2, and 40K SE2 have substantially no agglutination activity against both rabbit and human erythrocytes. 5K SE2 exhibited an activity with about a quarter of that of stabilized galectin 9.
That is, the obtained results were as follows:

40K SE2, 20K SE2, 10K SE2: inactive,
and
5K SE2 < stabilized galectin.

Example 4

Efficacy of PEGylated stabilized galectin 9 on CIA model rats

1. Method

Bovine type II collagen (Collagen Gizyutsu Kensyu-kai (Collagen Technical Research), Japan) was dissolved in incomplete Freund's Adjuvant (IFA: Difco). One milliliter of the resulting emulsion (collagen 800 μg/1 mL/rat) was subcutaneously injected into the back of each rat (six-week old Lewis female: Nippon SLC, Japan). One week after the injection, the same collagen emulsion solution (collagen 800 μg/0.3 mL/rat) was administered to the rat via the base of the tails, as secondary immunization. Seven days after the secondary immunization, each PEGylated modified galectin 9 protein (PEG-Gal9-10K (PEG-Stable Gal9(10K)), PEG-Gal9-20K (PEG-Stable Gal9(20K)), or PEG-Gal9-40K (PEG-Stable Gal9(40K)), 0.6 mg/kg) was intravenously administered to the rats three times per week for 31 days. Volumes of both hind legs of the rats were measured on the first collagen immunization day (0 day) and three times per week (Mon., Wed., and Fri.) after the first immunization, and swelling ratios (%) were calculated the following equation.
Swelling ratio (%) = [(paw volume (mL) after immunization) - (paw volume (mL) before immunization)]/[paw volume (mL) before immunization] × 100

Statistical analysis was conducted as follows. The average swelling ratio (%) and the standard error were determined using the total volume of both hind legs as an individual value. Difference between the PBS group and the

PEGylated modified galectin 9 protein group was evaluated by a Dunnett's Multiple Comparison Test or a Bonferroni Post-Test using commercially available statistical software (GraphPad Software, Inc., San Diego, USA). Every significant level was set at less than 5% ($p < 0.05$), and data were shown with significance at less than 5% and less than 1% ($p < 0.01$).

2. Results

The obtained results are shown in FIG. 7. PEGylated stabilized galectin 9 (PEGylated modified galectin 9 protein) was recognized to have therapeutic efficacy on CIA (collagen-induced arthritis). Since therapeutic effectiveness for a system used as a human chronic rheumatoid arthritis model was observed, PEGylated stabilized galectin 9 (PEGylated modified galectin 9 protein) was recognized to serve effectively as a therapeutic agent for chronic rheumatoid arthritis. Similarly, PEGylated galectin 9 and PEGylated modified galectin 9 proteins can be expected to be an anti-inflammatory agent, an anti-allergy agent, or an immunosuppressive agent.
As a result of testing the performance of the stabilized galectin 9 conjugated with PEG (5K, 10K, 20K, or 40K) (PEGylated stabilized galectin 9), it was recognized that the binding of PEG elongated the blood elimination half-life of the stabilized galectin 9 and enhanced the proliferation-inhibiting activity against PC-3 (prostatic cancer) cells and MOLT-4 cells and eliminated the hemagglutination activity. Thus, the PEG binding was recognized to be excellent in increase of solubility and blood elimination half-life of stabilized galectin 9. Furthermore, in some cases, hemagglutination activity was eliminated, and it was recognized that this elimination of hemagglutination was proportional to the length of the PEG chain bound to stabilized galectin 9. Since hemagglutination activity, which may become adverse effect when galectin 9 or stabilized galectin 9 (modified galectin 9 protein) is administered in vivo, can be eliminated by PEGylation, it is obvious that PEGylated galectin 9 and PEGylated stabilized galectin 9 (PEGylated modified galectin 9 protein) are materials having enhanced usefulness as a therapeutic agent.

INDUSTRIAL APPLICABILITY

The galectin 9-polymer conjugate of the present invention is an excellent candidate as a drug. In particular, PEGylated stabilized galectin 9 (PEGylated modified galectin 9 protein) is a material having enhanced usefulness as a therapeutic agent, wherein proliferation-inhibiting activity (or cytotoxicity) against cancer cells is enhanced, and undesirable activities, such as hemagglutination activity, are decreased or eliminated. PEGylated stabilized galectin 9 can be developed into an anti-tumor agent, an anticancer agent, an anti-inflammatory agent, an anti-allergy agent, an immunosuppressive agent, an anti-virus agent, or the like and can be used as a reagent for functional investigation of galectin 9.
While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

**1.** A chemically modified protein derivative of galectin 9 or a modified galectin 9 protein, which is a galectin 9-polymer conjugate wherein a polymer is bound, through a covalent bond, to a member selected from the group consisting of galectin 9 and modified galectin 9 proteins.

**2.** The chemically modified protein derivative according to Claim 1, wherein the polymer is a polyalkylene oxide or an alkoxy-terminated polyalkylene oxide.

**3.** The chemically modified protein derivative according to Claim 1 or 2, wherein the polymer is a poly(ethylene glycol) (PEG) or a lower alkoxy-terminated poly(ethylene glycol).

**4.** The chemically modified protein derivative according to any of Claims 1 to 3, wherein the polymer is a monomethoxy-poly(ethylene glycol) (mPEG).

**5.** The chemically modified protein derivative according to any of Claims 1 to 4, wherein the polymer has a molecular weight of approximately 200 to 100000.

**6.** The chemically modified protein derivative according to any of Claims 1 to 5, wherein the polymer has a molecular weight of approximately 5000 to 40000.

7. The chemically modified protein derivative according to any of Claims 1 to 6, which is prepared by reacting mPEG-SPA or mPEG2-NHS with a member selected from the group consisting of galectin 9 and modified galectin 9 proteins.

8. The chemically modified protein derivative according to any of Claims 1 to 7, wherein the member selected from the group consisting of galectin 9 and modified galectin 9 proteins is G9NC(null).

9. A drug, which comprises a chemically modified protein derivative of galectin 9 or a modified galectin 9 protein that is a galectin 9-polymer conjugate wherein a polymer is bound, through a covalent bond, to a member selected from the group consisting of galectin 9 and modified galectin 9 proteins.

10. The drug according to Claim 9, which comprises a mixture of positional isomers of a polymer conjugate of a member selected from the group consisting of galectin 9 and modified galectin 9 proteins.

11. The drug according to Claim 9 or 10, which serves as an agent selected from the group consisting of anti-tumor agents (anticancer agents), anti-allergy agents, immunosuppressive agents, anti-autoimmune disease agents, anti-inflammatory agents, and adrenocortical steroid hormone substitutes.

12. A process for producing a chemically modified protein derivative of galectin 9 or a modified galectin 9 protein, which comprises the step of:

reacting a member selected from the group consisting of galectin 9 and modified galectin 9 proteins with a PEGylating reagent under conditions whereby a covalent bond for PEGylating protein is formed to provide a PEGylated galectin 9 conjugate.

13. The process according to Claim 12, wherein the PEGylating reagent is carbonyloxy-N-dicarboximide.

14. The process according to Claim 12 or 13, wherein the PEGylating reagent is mPEG-SPA or mPEG2-NHS.

【FIG. 1】

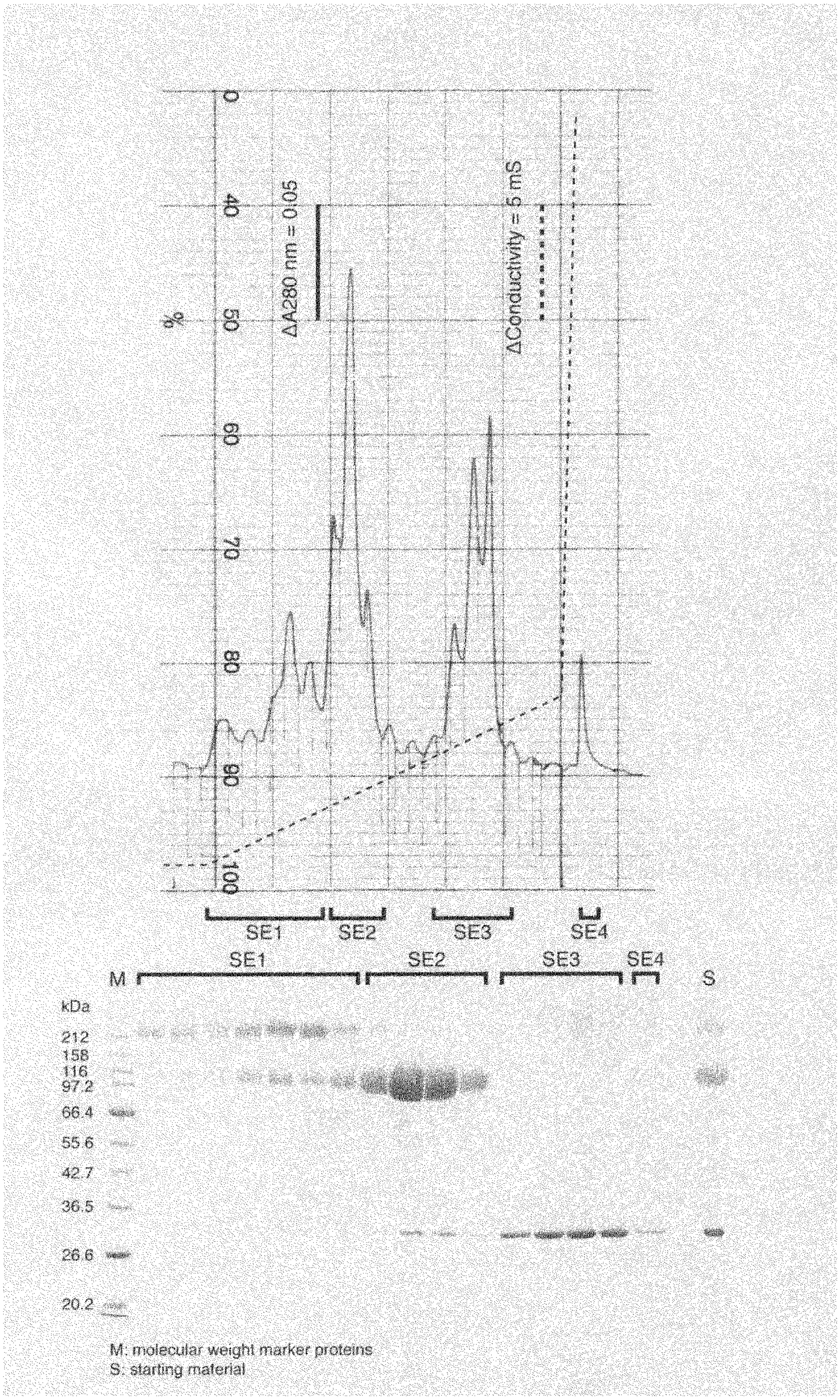
%
ΔA280 nm = 0.05
ΔConductivity = 5 mS
SE1
SE2
SE3
SE4
M
S
kDa
212
158
116
97.2
66.4
55.6
42.7
36.5
26.6
20.2
M: molecular weight marker proteins
S: starting material

【FIG. 2】

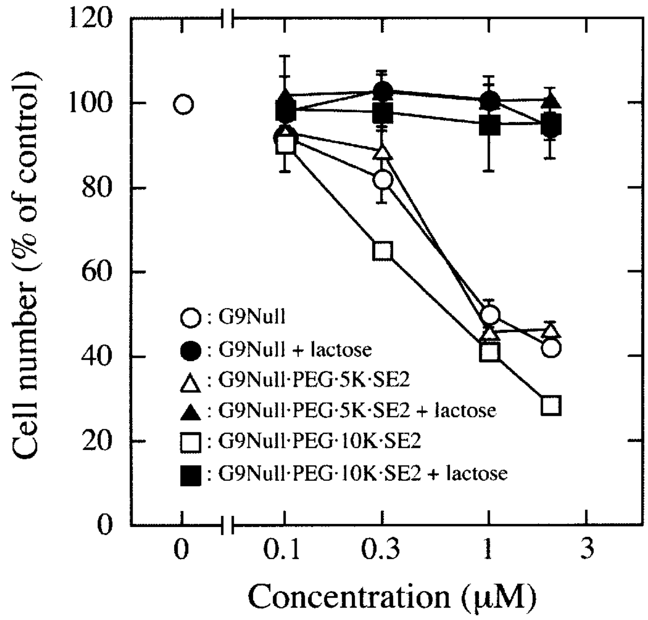
Cell number (% of control)
120
100
80
60
40
20
0
0
0.1
0.3
1
3
Concentration (μM)
○ : G9Null
● : G9Null + lactose
△ : G9Null·PEG·5K·SE2
▲ : G9Null·PEG·5K·SE2 + lactose
□ : G9Null·PEG·10K·SE2
■ : G9Null·PEG·10K·SE2 + lactose

【FIG. 3】

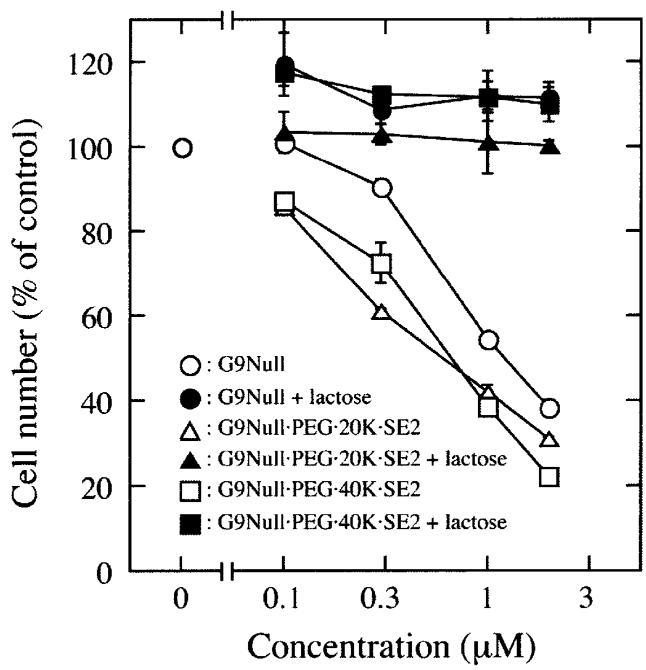
Cell number (% of control)
120
100
80
60
40
20
0
0
0.1
0.3
1
3
Concentration (μM)
○ : G9Null
● : G9Null + lactose
△ : G9Null·PEG·20K·SE2
▲ : G9Null·PEG·20K·SE2 + lactose
□ : G9Null·PEG·40K·SE2
■ : G9Null·PEG·40K·SE2 + lactose

【FIG. 4】

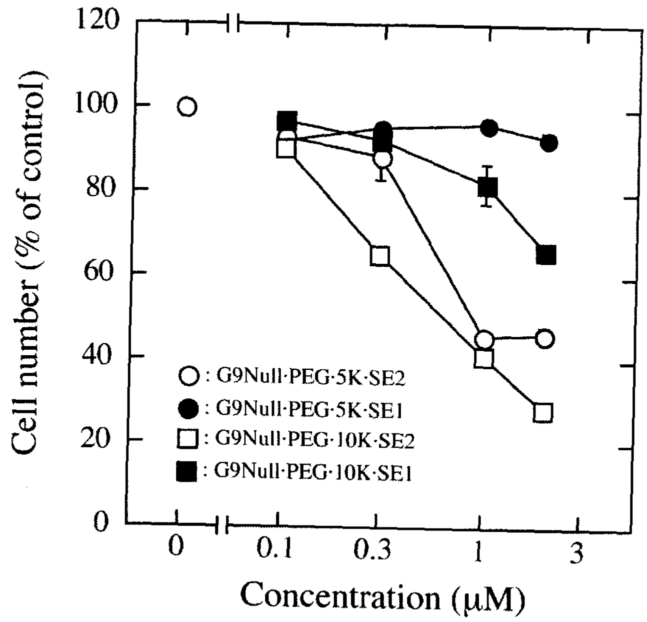
120
100
80
60
40
20
0
Cell number (% of control)
○ : G9Null·PEG·5K·SE2
● : G9Null·PEG·5K·SE1
□ : G9Null·PEG·10K·SE2
■ : G9Null·PEG·10K·SE1
0
0.1
0.3
1
3
Concentration (μM)

【FIG. 5】

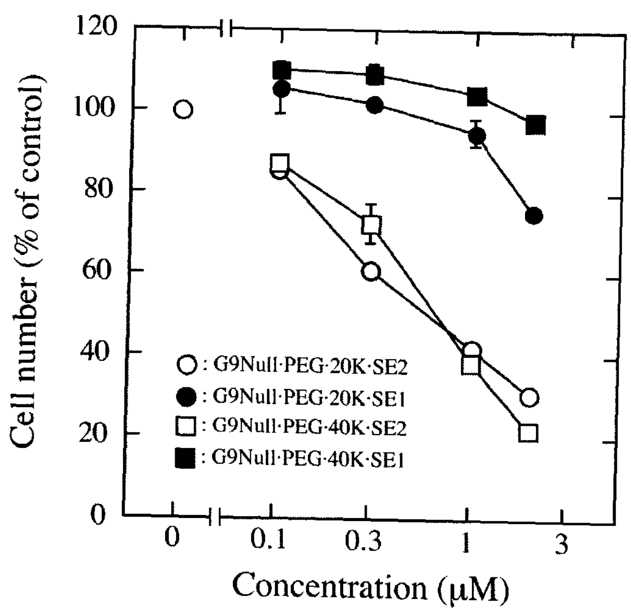
120
100
80
60
40
20
0
Cell number (% of control)
○ : G9Null·PEG·20K·SE2
● : G9Null·PEG·20K·SE1
□ : G9Null·PEG·40K·SE2
■ : G9Null·PEG·40K·SE1
0
0.1
0.3
1
3
Concentration (μM)

【FIG. 6】

FIG. 6

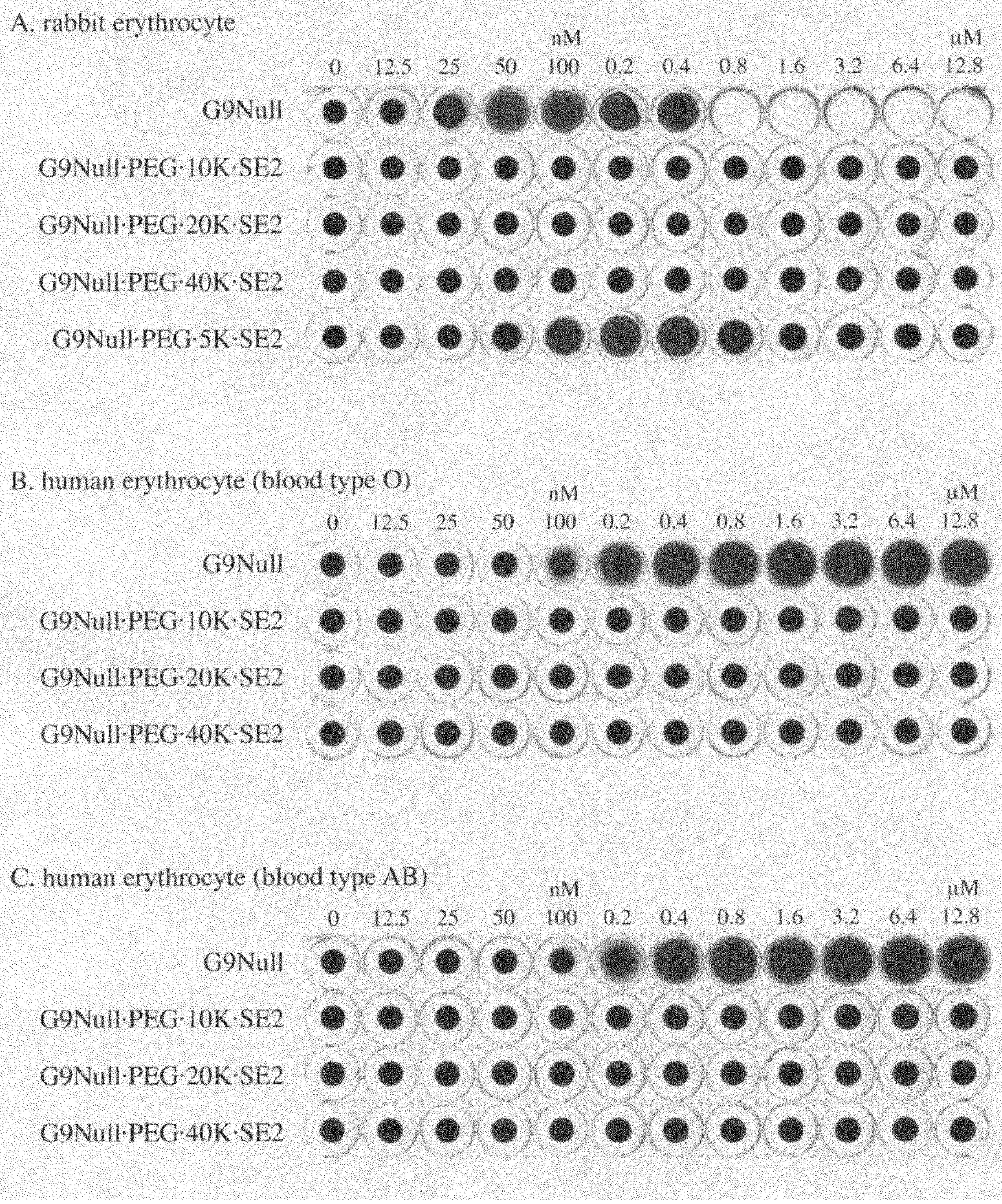
A. rabbit erythrocyte
nM
μM
0 12.5 25 50 100 0.2 0.4 0.8 1.6 3.2 6.4 12.8
G9Null
G9Null·PEG·10K·SE2
G9Null·PEG·20K·SE2
G9Null·PEG·40K·SE2
G9Null·PEG·5K·SE2
B. human erythrocyte (blood type O)
nM
μM
0 12.5 25 50 100 0.2 0.4 0.8 1.6 3.2 6.4 12.8
G9Null
G9Null·PEG·10K·SE2
G9Null·PEG·20K·SE2
G9Null·PEG·40K·SE2
C. human erythrocyte (blood type AB)
nM
μM
0 12.5 25 50 100 0.2 0.4 0.8 1.6 3.2 6.4 12.8
G9Null
G9Null·PEG·10K·SE2
G9Null·PEG·20K·SE2
G9Null·PEG·40K·SE2

【FIG. 7】

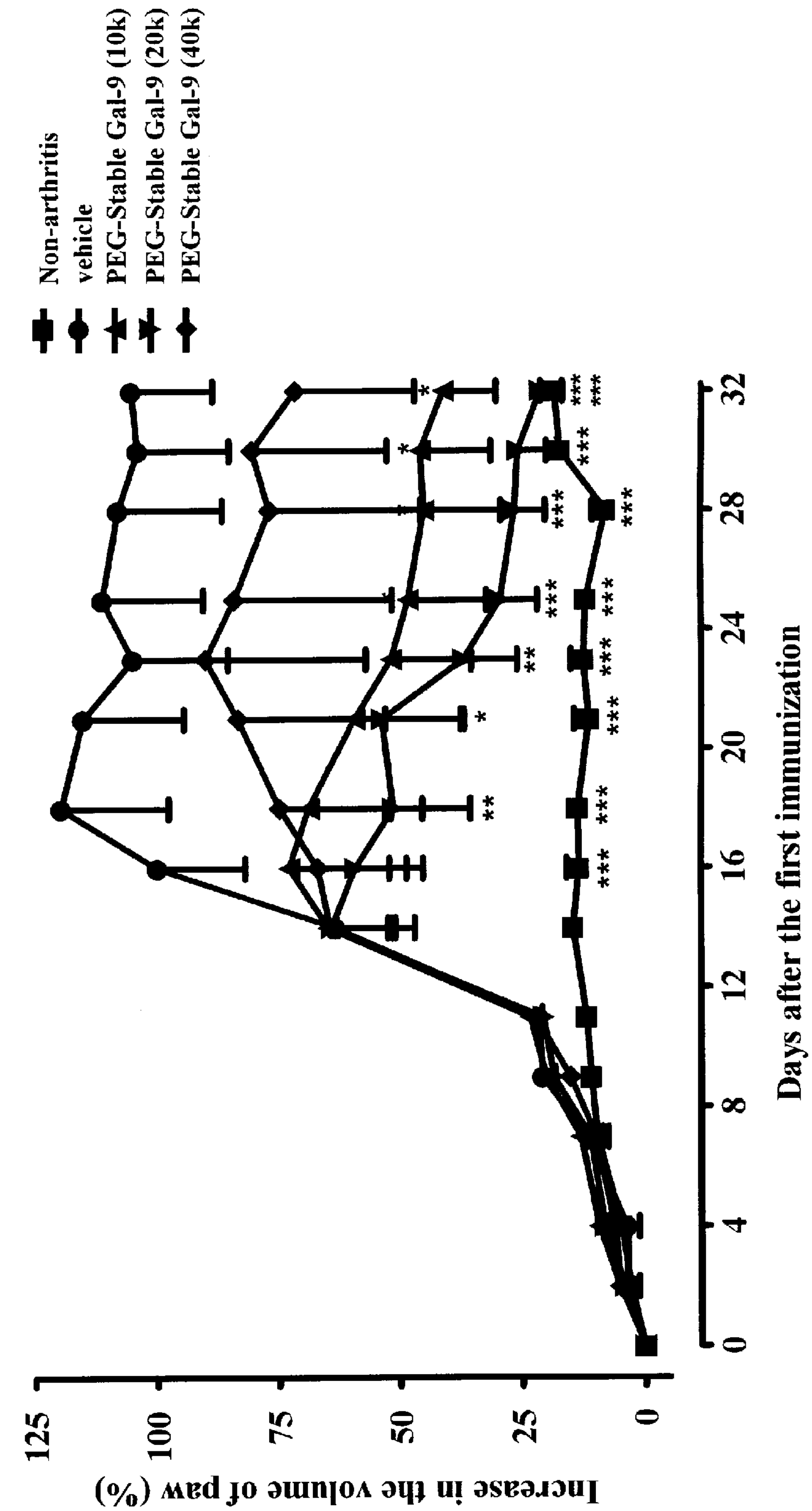
Non-arthritis
vehicle
PEG-Stable Gal-9 (10k)
PEG-Stable Gal-9 (20k)
PEG-Stable Gal-9 (40k)
Increase in the volume of paw (%)
0
25
50
75
100
125
Days after the first immunization
0
4
8
12
16
20
24
28
32

## INTERNATIONAL SEARCH REPORT

International application No.
PCT/JP2007/064144

A. CLASSIFICATION OF SUBJECT MATTER
*C07K14/42*(2006.01)i, *A61K38/00*(2006.01)i, *A61P29/00*(2006.01)i, *A61P35/00*(2006.01)i, *A61P37/06*(2006.01)i, *A61P37/08*(2006.01)i

According to International Patent Classification (IPC) or to both national classification and IPC

B. FIELDS SEARCHED

Minimum documentation searched (classification system followed by classification symbols)
C07K14/42, A61K38/00, A61P29/00, A61P35/00, A61P37/06, A61P37/08

Documentation searched other than minimum documentation to the extent that such documents are included in the fields searched

Electronic data base consulted during the international search (name of data base and, where practicable, search terms used)
BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| X | JP 2004-244411 A (Galpharma Co., Ltd.), 02 September, 2004 (02.09.04), Particularly, page 38; Par. No. [0083] & WO 2004/064857 A1 & EP 1586325 A1 & US 2006/0134119 A1 | 1-14 |
| A | JP 2002-541832 A (HUMAN GENOME SCIENCES, INC.), 10 December, 2002 (10.12.02), Particularly, page 55, Par. No. [0104] to page 57, Par. No. [0108] & WO 2000/063221 A2 & EP 1192168 A1 | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 August, 2007 (23.08.07) | 04 September, 2007 (04.09.07) |
| Name and mailing address of the ISA/ Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04064857 A
- WO 05093064 A
- JP 56023587 A
- WO 0237114 A1
- JP 2005006580 W
- WO 02037114 A


### Non-patent literature cited in the description


- **TURECI O. et al.** *J. Biol. Chem.,* 1997, vol. 272 (10), 6416-22
- **MATSUMOTO R. et al.** *J. Biol. Chem.,* 1998, vol. 273, 16976-84
- **MATSUSHITA N. et al.** *J. Biol. Chem.,* 2000, vol. 275, 8355-60
- *J. Biol. Chem.,* 2000, vol. 275 (12), 8355-8360
- **J. SAMBROOK et al.** Molecular Cloning: A Laboratory Manual. Cold Spring Harbor Laboratory Press, 1989
- DNA Cloning. The Practical Approach Series. IRL Press, Oxford University Press, 1995, vol. 1-4
- Zoku-Seikagaku Jikken Kouza 1, Idenshi Kenkyuho II. Nippon Seikagakukai. Tokyo Kagaku Dojin, 1986
- Shin-Seikagaku Jikken Kouza 2, Kakusan III. Nippon Seikagaku Kai. Tokyo Kagaku Dojin, 1992
- Oligonucleotide Synthesis. IRL Press, 1984
- Nucleic Acid Hybridization, A Practical Approach. IRL Press Ltd, 1985
- Transcription and Translation: A Practical Approach. IRL Press Ltd, 1984
- **B. PERBAL.** A Practical Guide to Molecular Cloning. John Wiley & Sons, 1988
- Gene Transfer Vectors for Mammalian Cells. Cold Spring Harbor Laboratory, 1987
- Immunochemical Methods in Cell and Molecular Biology. Academic Press, 1987
- Protein Purification: Principles and Practice. Springer-Verlag, 1987
- Handbook of Experimental Immunology. Blackwell Scientific Publications, 1986, vol. 1, 2, 3,
- Weir's Handbook of Experimental Immunology. Blackwell Science Ltd, 1997, vol. 1, 2, 3,
- Vaccines new approaches to immunological problems. 1992
- Methods in Enzymology. Academic Press, 1980, vol. 68
- Methods in Enzymology. vol. 100
- METHODS IN ENZYMOLOGY. Academic Press, 1983, vol. 101
- Methods in Enzymology. vol. 153
- METHODS IN ENZYMOLOGY. vol. 154
- METHODS IN ENZYMOLOGY. Academic Press, vol. 155
- Methods in Enzymology. Academic Press, 1991, vol. 204
- Methods in Enzymology. Academic Press, 1993, vol. 218
- Methods in Enzymology. Academic Press, 1999, vol. 303
- Methods in Enzymology. Academic Press, 1999, vol. 306
- Methods in Enzymology. Academic Press, 2000, vol. 326-328
- Methods in Enzymology. Academic Press, 2005, vol. 392
- **NOWAK et al.** *Biochem. Biophys. Res. Commun,* 1976, vol. 68, 650-657
- **NOWAK et al.** *Biochem. Biophys. Res. Commun.,* 1976, vol. 68, 650-657